# EUROPEAN PATENT SPECIFICATION

(11) **EP 2 495 235 B1**
(45) Date of publication and mention of the grant of the patent: **05.08.2015**
(21) Application number: 11157027.1
(22) Date of filing: 04.03.2011
(51) Int. Cl.: C07C 405/00, C07D 307/935

(54) **Process for the synthesis of prostaglandins and intermediates thereof**
Herstellungsverfahren zur Synthese von Prostaglandinen und Zwischenprodukte davon
Procédé de synthèse de prostaglandines et intermédiaires correspondants

(43) Date of publication of application: 05.09.2012
(73) Proprietor: Newchem S.p.A., 20123 Milano (IT)
(72) Inventor: Mariani, Edoardo, 20123 Milano (IT); Orru', Gianmauro, 20123 Milano (IT); Montorsi, Mauro, 20123 Milano (IT); Andriolo, Erika, 20123 Milano (IT); Bandini, Marco, 40126 Bologna (IT); Contento, Michele, 40126 Bologna (IT); Tolomelli, Alessandra, 40126 Bologna (IT)
(74) Representative: Banfi, Paolo

(56) References cited:
- EP-A1- 0 364 417
- EP-B1- 0 544 899
- WO-A1-2010/097672
- US-A1- 2009 259 066
- DEPRE D ET AL: "A short multigram asymmetric synthesis of prostanoid scaffolds", TETRAHEDRON, ELSEVIER SCIENCE PUBLISHERS, AMSTERDAM, NL, vol. 59, no. 35, 25 August 2003 (2003-08-25), pages 6797-6812, XP004448490, ISSN: 0040-4020, DOI: DOI:10.1016/S0040-4020(03)00920-7
- MULZER AND CO: LIEBIGS ANNALEN DER CHEMIE, 13 June 1994 (1994-06-13), pages 531-539, XP002652796,
- ZANONI G ET AL: "The Meyer-Schuster rearrangement: a new synthetic strategy leading to prostaglandins and their drug analogs, Bimatoprost and Latanoprost", TETRAHEDRON, ELSEVIER SCIENCE PUBLISHERS, AMSTERDAM, NL, vol. 66, no. 38, 18 September 2010 (2010-09-18), pages 7472-7478, XP027231278, ISSN: 0040-4020 [retrieved on 2010-08-01]
- SEONG-WOO HWANG ET AL: "TOTAL SYNTHESIS OF 12-EPI-PGF2", TETRAHEDRON LETTERS, ELSEVIER, AMSTERDAM, NL, vol. 37, no. 6, 5 February 1996 (1996-02-05), pages 779-782, XP004030259, ISSN: 0040-4039, DOI: DOI:10.1016/0040-4039(95)02390-9
- FRIGERIO AND CO: TETRAHEDRON LETTERS, vol. 35, no. 43, 1 January 1994 (1994-01-01), pages 8019-8022, XP002652797,

## Description

A process is disclosed for the preparation of the prostaglandins of the PGF_{2α}-series.

The invention relates to a method for the preparation of the prostaglandin F_{2α}-series including in particular Latanoprost, Bimatoprost and Travoprost which are active in the treatment of ocular hypertensive conditions and glaucoma.

### Known art

_{2α} prostaglandin synthetic routes so far reported may comprise the following two major steps:

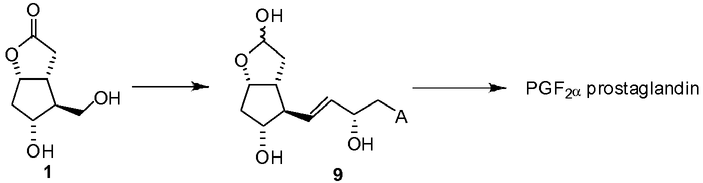

The starting compound is generally protected on the secondary hydroxyl group: 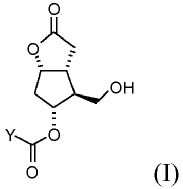 where Y stands for *p*-phenylbenzoyl (PPB) (Ia) or benzoyl (Bz) (Ib) or an analogous substituted aryl group.

For example, EP0364417B1 (Kabi Pharmacia AB) describes the following synthetic sequence:

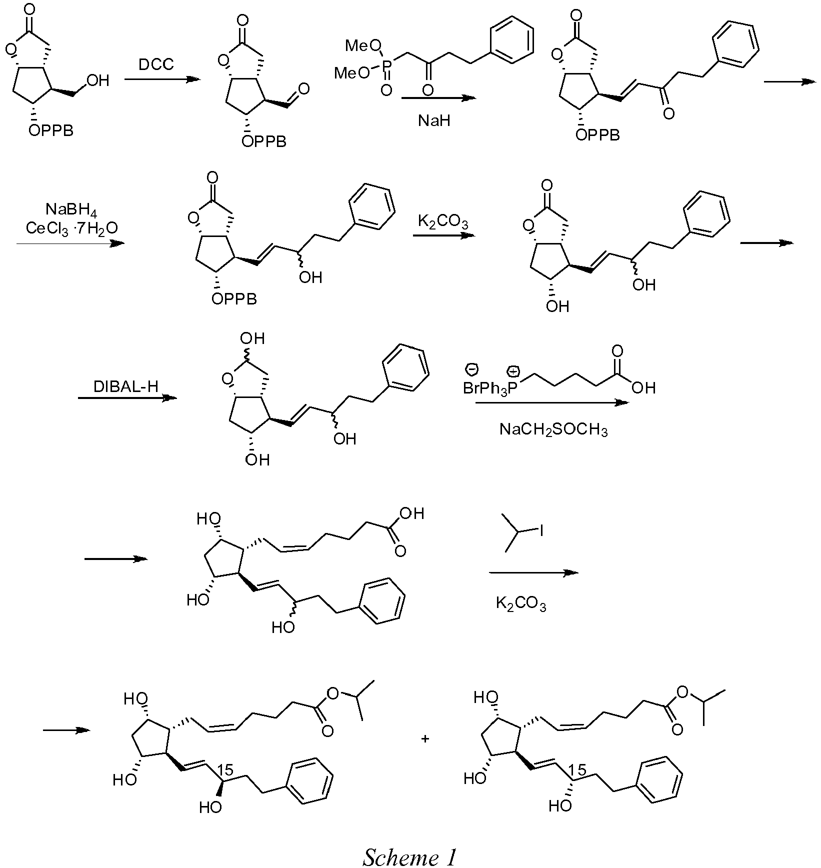

The product is obtained as a mixture of epimers where the 15-OH can be in α-position or β-position. The removal of unwanted β-isomer and of other impurities represents one of the main difficulties in the preparation of prostaglandins and many methods have been proposed to reduce the amount of the undesired diastereoisomers formed during the preparation.

In EP0544899B1 (Pharmacia AB) the reduction of the α,β-unsaturated ketone is performed with lithium-tri(*sec*-butyl)borohydride at -130°C.

In this kind of processes the low selectivity in the reduction of the keto group leads to a tedious separation of the diastereomers and generally decreases the global yield of the synthesis.

A different method of stereoselective reduction of the ketone is proposed in US7674921 B1 (Cayman Chemical Co.) where the reaction with lithium aluminium hydride in the presence of (S)-binaphtol in tetrahydrofuran at -78°C is reported.

An alternative is presented in US6927300B2 and in US 7157590B2 (Finetech Lab. Ltd.) where the critical step of the reduction of the α,β-unsaturated keto-group is performed on the compound reported below 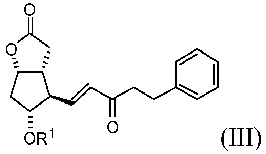 where R¹ is an aryl carbonyl group in the presence of (-)β-chlorodiisopinocampheylborane in tetrahydrofuran at -25°C which allows to obtain a ratio of 95/5 of the two diastereoisomers (R)-(IV)/(S)-(IV).

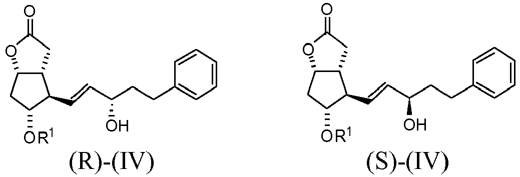

A further improvement in the preparation of PGF_{2α} is directed to the reduction of lactone to lactol in order to introduce the α-chain by subsequent Wittig reaction with 4-carboxybutyltriphenylphosphonium bromide.

The reaction was usually carried out using diisobutylaluminum hydride at very low temperatures, namely between -80°C and -40°C. Unfortunately, in these conditions the partial removal of the protecting group PPB or benzoyl (Bz) is possible.

To avoid the inconvenient of working with mixtures of products, the *p*-phenylbenzoyl group or the benzoyl group R is removed by basic hydrolysis and a suitable protecting group is introduced at the two hydroxyl groups of the so obtained compound according to the following scheme:

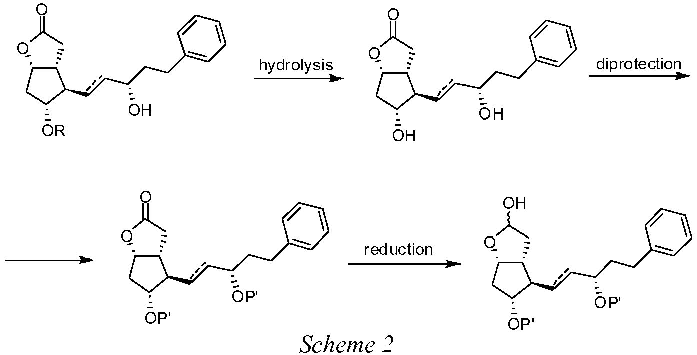

In this way two additional steps of protection and deprotection are introduced possibly leading to a decrease of the global yield of the process at an advanced stage of the synthesis.

Moreover when P' is a silylated group as reported in US7268239B2 (Resolution Chemical Ltd.), a mixture of products is obtained because of the migration of the silyl group as shown in scheme 3

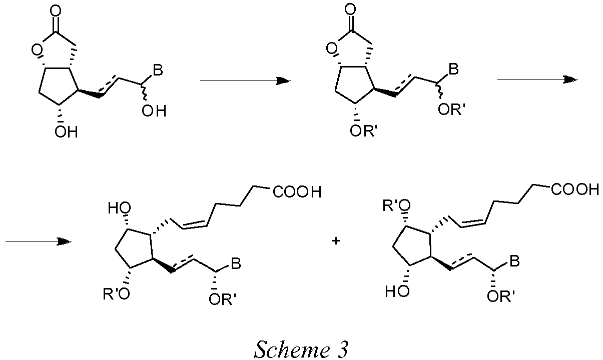

The same approach of hydrolysis of the aroyl group R followed by diprotection of the two hydroxyl groups with trialkylsilyl group or triaryl silyl group or tetrahydropiranyl group is disclosed in US7642370B2 (Daichii Fine Chemical Co.). The protection of the two hydroxyl groups coming before the reduction of the lactone ring to lactol is described also in US7674921B1 (Cayman Chemical Co.) and in US6689901B2 (Pharmacia and Upjohn Company).

It follows that one important issue in the synthesis of PGF_{2α} is the involvement of intermediates with the most appropriate hydroxyl protecting group. As a consequence the use of the starting Corey lactone carrying a protection able to survive to the conditions of the subsequent reactions allows to form intermediates easy to isolate and to purify. Moreover the protection should be selectively removed in mild conditions.

In US5359095 (Pharmacia AB) the preparation of PGF_{2α} prostaglandins is described starting from the commercially available (-)-Corey lactone (Ia) which is oxidized to the corresponding aldehyde (IIa)

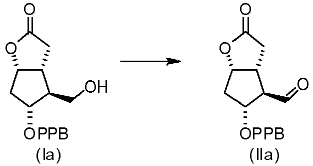

The reaction is carried out in the presence of dicyclohexylcarbodiimide in dimethylsulfoxide and 1,2-dimethoxyethane; after quenching with orthophosphoric acid the aldehyde is obtained. In the same application it is reported that the crude aldehyde (II) is particularly unstable and must be used within a short period after preparation.

In US2010/0010239A1 (Sandoz AG) compound (I) is preferably oxidized in dichloromethane with oxalyl chloride and DMSO; the aldehyde (II) is not isolated and is processed directly in the solution where it is obtained or, when necessary, stored in solution at a temperature between -20 and 0°C.

In US7268239B2 (Resolution Chem. Ltd.) the aldehyde (II) 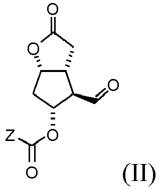 where Z is (C₆-C₁₀)-aryl optionally substituted with one to three substituents independently selected from the group consisting of halo, C₁ to C₆ alkyl and unsubstituted C₆ to C₁₀ aryl, is formed by oxidation of the alcohol with sodium hypochlorite and 2,2,6,6-tetramethtyl-1-piperidinyloxy free radical (TEMPO); in order to avoid the risk of degradation, the aldehyde is directly used in the organic solution where it is synthesized.

According to US2009/0287003A1(Eastar Chem. Corp.) five steps can be performed to prepare the aldehyde to be used as starting material for the synthesis of latanoprost. The five steps are reported in the scheme below:

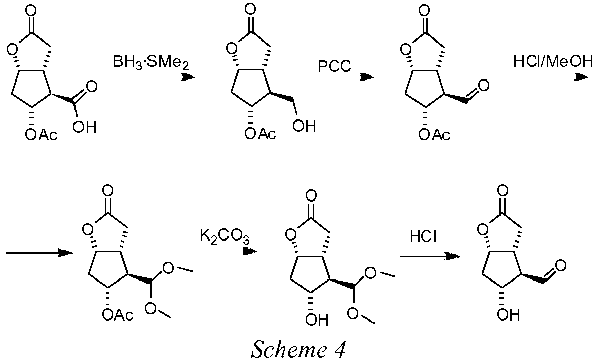

In WO2010/097672 (Sifavitor) the starting material is the Corey lactone where the hydroxyl group is protected as *tert*-butyldimethyl silyl derivative and during the synthesis a second protection is introduced according the scheme reported below referred to the preparation of Bimatoprost:

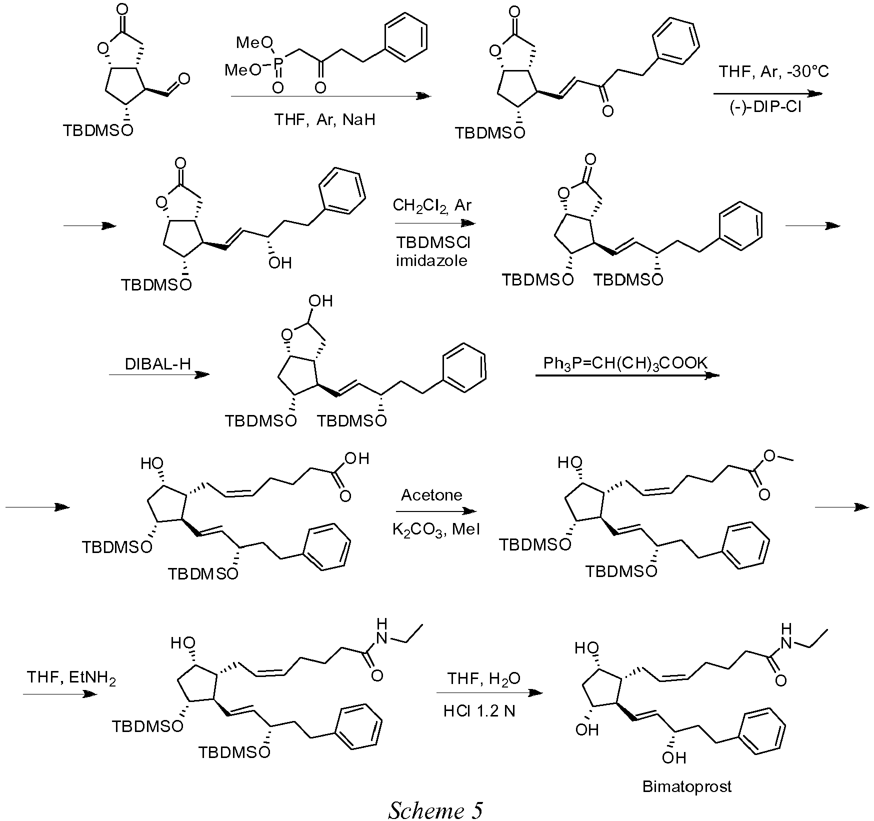

### Description of the invention

Object of the present invention is a process for the preparation of PGF_{2α} derivatives of formula (I) 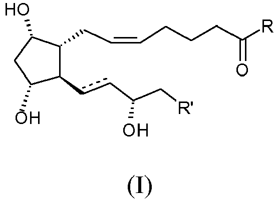 wherein:
1) R is (CH₃)₂CHO-, R' is benzyl and ---- in formula (I) is a single bond,
2) R is (CH₃)₂CHO-, R' is (3-trifluoromethyl)benzyloxy and ---- in formula (I) is either a single or a double bond
3) R is CH₃CH₂NH-, R' is benzyl and ---- in formula (I) is either a single or a double bond,
according to the following reaction scheme: 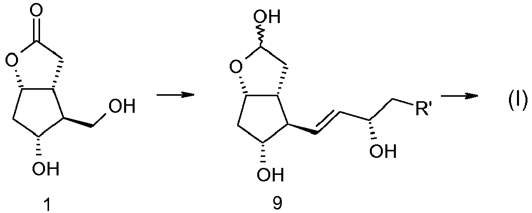 wherein the commercially available Corey diol 1 is converted into compound **9** through the following steps: 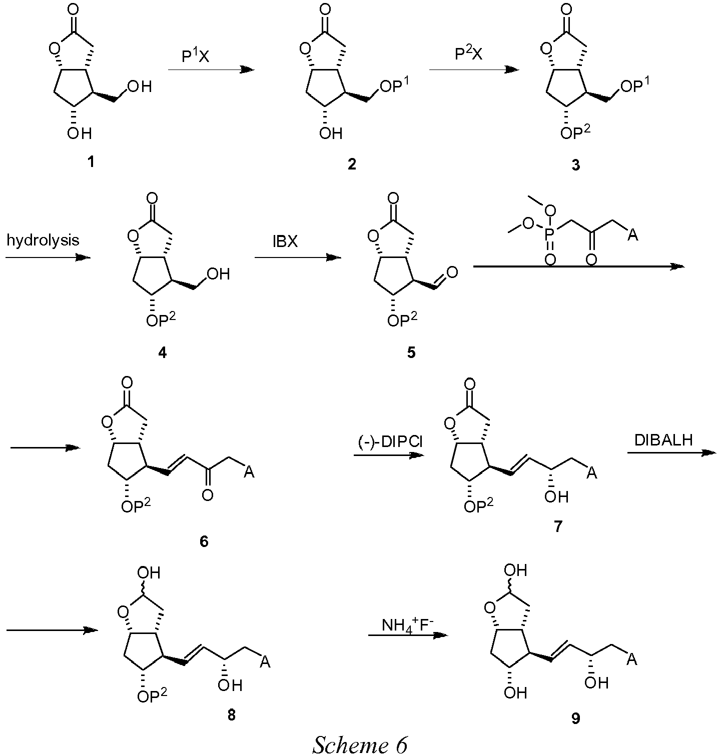 wherein
P¹ = triarylmethyl optionally substituted on the aryl moiety; -SiQ¹₃ wherein Q¹ independently from one another represents (C₁-C₄)alkyl, (C₆-C₁₀)aryl, (C₁-C₄)alkyl(C₆-C₁₀)aryl or (C₁-C₄)alkoxy(C₆-C₁₀)aryl
P² = -SiQ²₂Q³ wherein Q² = (C₆-C₁₀)aryl and Q³ = (C₁-C₄)alkyl with the condition that P¹≠P²
or P¹ and P² are selected from the following pairs:
P¹ = *tert*-butyldimethylsilyl- and P² = *tert*-butyldiphenylsilyl-
P¹ = triphenylmethyl- and P² = *tert*-butyldiphenylsilyl-
P¹ = triphenylmethyl- and P² = triphenylsilyl-
X = halogen, -OSO₂CF₃
A = benzyl or (3-trifluoromethyl)phenoxy
(-)-DIPCl =(-)-diisopinocampheylborane
DIBALH= diisobutylaluminium hydride
IBX= 2-iodoxybenzoic acid

In the above invention process, compound **1** is converted first to the monoprotected diol **2** and then to diprotected diol **3** according to scheme 7

After removal of the primary hydroxyl protecting group P¹ to form compound **4**, oxidation is carried out to prepare aldehyde **5**.

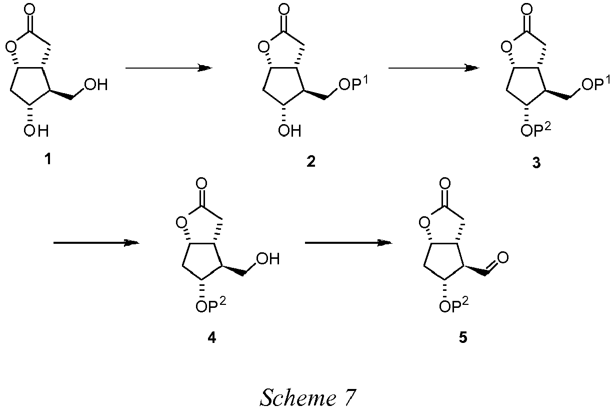

The aldehyde **5** is prepared by introducing a hydroxy protecting group in order to form stable subsequent intermediates and avoiding mixtures of products difficult to handle. Compared to the intermediate compound used in the process of EP364417 (an aldehyde protected at the secondary-hydroxy group with *p*-phenylbenzoyl which is known to be unstable in solution and to undergo partial decomposition during work-up), compound **5** is more stable and thanks to the presence of a bulky protecting group, allows to dispense with a second hydroxyl protection of intermediate **7** introduced in subsequent reaction steps. Compound **5,** as well as the method for its preparation according to the scheme 7 above, are not part of the present invention.

The aldehyde **5** is then subjected to reaction with dimethyl 2-oxo-4-phenylbutylphosphonate to prepare Latanoprost and Bimatoprost or with dimethyl 2-oxo-3-(3-(trifluoromethyl)phenoxy)propylphosphonate to prepare Travoprost, thereby obtaining intermediate **6** 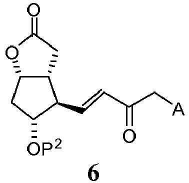 wherein A is benzyl or 3-(trifluoromethyl)phenoxy.

The oxo group of the side chain is then reduced to obtain compound **7**

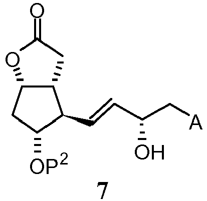

The lactone is subsequently reduced to lactol **8**

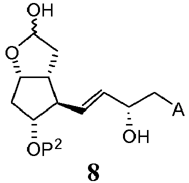

The protecting group P² is hydrolyzed to form compound **9**

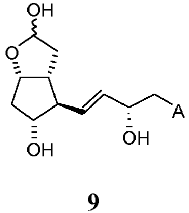

In a preferred embodiment of the invention, the protecting groups P¹ and P² are selected from the following pairs:
P¹ = *tert*-butyldimethylsilyl- (TBDMS) and P² = *tert*-butyldiphenylsilyl-(TBDPS);
P¹ = triphenylmethyl- (Tr) and P² = *tert*-butyldiphenylsilyl-;
P¹ = triphenylmethyl- and P² = triphenylsilyl- (TPS);

In another preferred embodiment, the conversion of compound **1** into compound 3 with P¹= TBDMS and P²= TBDPS is a one-pot process carried out in *N*,*N*-dimethylformamide as a solvent by reaction first with *tert*-butyldimethylsilyl chloride and imidazole at a temperature between -15 and 5°C until complete conversion to **2**, followed by direct addition into the reaction mixture of a second portion of imidazole and *tert*-butyldiphenylsilylchloride at a temperature between 0°C and 25°C.

In an embodiment which is not part of the present invention, the oxidation of compound **4** to compound **5** is carried out with IBX at a temperature ranging from 0°C to 40°C with a molar ratio between IBX and compound **4** from 1 to 4 in a solvent selected from dimethyl sulfoxide, *N,N-*dimethylformamide, tetrahydrofuran, methyltetrahydrofuran or a mixture thereof.

In case Bimatoprost is the end product, compound **9** with A=benzyl (in the following scheme, compound **9a**) is directly converted by Wittig reaction to the acid **10a** which is then converted in one step to ethylamide affording Bimatoprost, according to the following scheme:

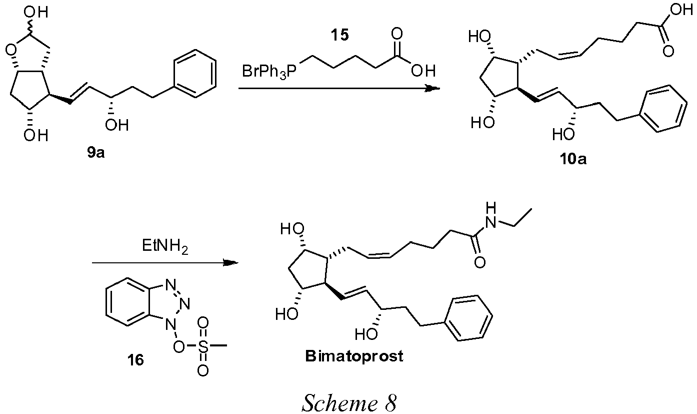

In a preferred embodiment, the conversion of compound **10a** to Bimatoprost is performed by reaction with ethylamine in an organic solvent, which is chosen among amides, ethers, ketones and chlorinated solvents, more preferably *N,N-*dimethylformamide, at a temperature between -35 and 25°C, more preferably between -20°C and -10°C, in the presence of triethylamine and a suitable coupling reagent, which is preferably 1-(methylsulfonyloxy)benzotriazole. The molar ratio between compound **10a** and ethylamine is comprised between 1 and 5, more preferably is 3.5, while the molar ratio between compound **10a** and the coupling reagent is comprised between 1 and 2.5, more preferably is 1.8.

When **Latanoprost** is the desired product the double bond on the side chain of compound **9a** is hydrogenated to form compound **11**, then by Wittig reaction with 4-carboxybutyltriphenylphosphonium bromide compound **11** is converted into Latanoprost acid **12**. By conversion of the carboxylic acid into isopropyl ester, the final product Latanoprost is obtained:

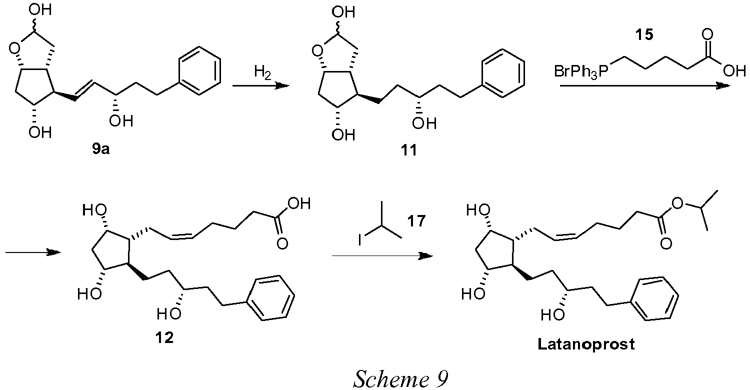

In the case of **Travoprost,** compound 9 with A=3-(trifluoromethyl)phenoxy (in the following scheme, compound **9b**) is converted into **10b**, which in turn is converted into Travoprost by esterification of the carboxylic acid by reaction with 2-iodopropane, according to scheme 10:

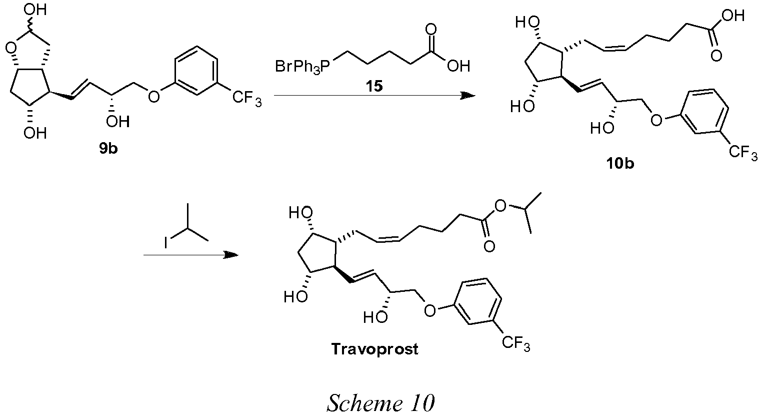

### Detailed disclosure of preferred embodiments

In the first step the Corey diol **1** is protected at the primary alcohol by reaction with *tert*-butyldimethylsilyl chloride (TBDMSCl) in the presence of imidazole as a base in *N*,*N*-dimethylformamide at a temperature between - 30°C and 10°C, more preferably between -15°C and 5°C. The reaction is highly selective and no protection at the secondary alcohol is detected when the reaction is performed in the conditions described above.

The protection of the secondary hydroxy-group is carried out in the presence of the same base and of *tert*-butyldiphenylsilyl chloride (TBDPSCl) affording compound **3** where P² = TBDPS- at a temperature between -20°C and 40°C, more preferably between 0°C and 25°C. The product obtained is used in the subsequent step without any purification.

The two protection steps of the primary and secondary hydroxy groups of the starting material **1** can easily be carried out one-pot.

The removal of TBDMS-group is obtained by action of diluted sulfuric acid in methanol at 25°C.

The yield of the first two steps is greater than 93%.

The alcohol **4** is converted into aldehyde **5** by oxidation with 2-iodoxybenzoic acid (IBX) (molar ratio IBX/aldehyde = 1.6) in dimethylsulfoxide at 25°C(this step is not part of the present invention); the aldehyde is obtained after work-up in quantitative yield.

The aldehyde shows a good stability and can easily be stored for at least 4-5 months at a temperature between -30°C and 0°C. This makes the compound easy to handle and allows more flexibility in the production planning.

In the synthesis of PGF_{2α} prostaglandins according to the invention, after preparation of the ylide of dimethyl 2-oxo-4-phenylbutylphosphonate or dimethyl 2-oxo-3-(3-(trifluoromethyl)phenoxy)propylphosphonate by treatment with triethylamine and lithium chloride in tetrahydrofuran at -10°C, the Horner-Wadsworth-Emmons reaction is performed by addition in situ of the aldehyde **5**. The product **6** is obtained after purification by column chromatography in 70% to 80% yield calculated from the alcohol **4**.

The reduction of the ketone is carried out by dissolving compound **6** in tetrahydrofuran and adding a 60% solution of (-)-β-chlorodiisopinocampheylborane in tetrahydrofuran. The reaction does not require a very low temperature and can be performed between -20°C and 10°C, preferably at about 0°C. The amount of reducing agent used is between 1 and 4 moles per mole of **6**; the excess of borane is then removed by quick filtration on silica gel. A purification by column chromatography is performed in order to remove all the inorganic impurities and the by-products, in particular compound **(R)-7** which would render very tedious and expensive the purification of the end products:

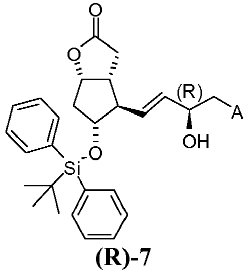

It has been then found that at this stage of the synthesis the reduction of the cyclic lactone can advantageously be performed by reaction of compound **7** with diisobutylaluminum hydride in toluene at about -78°C. After quenching the excess of reducing agent with methanol the work up affords the product **8** in very good yield (90%).

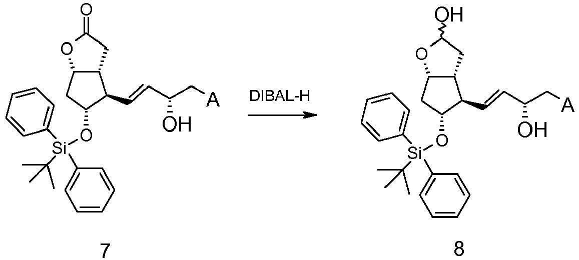

The removal of the protecting group TBDPS- is then performed by reaction of tetra-butylammonium fluoride in an organic solvent, preferably tetrahydrofuran, at a temperature between -20°C and +20°C, more preferably at 0°C. The yield of the two lactol isomers of compound **9** is about 90%.

Compound **9** represents the intermediate common to all the synthetic routes herein disclosed.

In the case of **Bimatoprost** the Wittig reaction is then performed to react 4-carboxybutyltriphenylphosphonium bromide by previous formation of the ylide in the presence of a base. Preferably the base is represented by potassium *tert*-butoxide and the reaction is carried out in tetrahydrofuran. The temperature of the reaction is comprised between -20°C and +20°C. The product is isolated by column chromatography and obtained in 91 % yield.

The formation of the amide to prepare the final product Bimatoprost is carried out in a single step by coupling of the acid **12** with ethylamine in the presence of a previously prepared coupling reagent according to Bulletin of the Chemical Society of Japan, 1978, 51(11), 3320-3329. The reaction is performed in an organic solvent, chosen among amides, ethers, ketones and chlorinated solvents. The solvent is preferably *N,N*-dimethylformamide, tetrahydrofuran, dioxane, acetone and dichloromethane; more preferably, the solvent is *N*,*N*-dimethylformamide. The temperature of the reaction is comprised between -35°C and 25°C, more preferably at -15°C. After acidic work-up the product is recrystallized from an organic solvent or a mixture of solvents. After recrystallization from dichloromethane/diisopropylether Bimatoprost is obtained in 55% yield.

In the case of the synthesis of **Latanoprost** an additional step is required to convert compound **9a** into compound **11**. More specifically the double bond has to be reduced to single bond by hydrogenation:

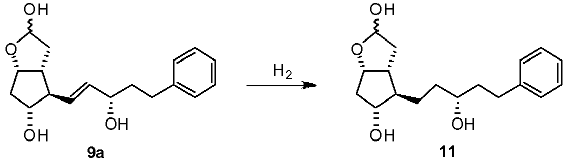

The reaction is performed suspending a catalyst, preferably 5% platinum on carbon, in an organic solvent, preferably in methanol. After the suspension is saturated with hydrogen for 2 to 10 hours, more preferable for 5 to 6 hours, compound **9a** is added as a solution in the same organic solvent, together with triethylamine. The intermediate **9a** obtained after filtration of the catalyst and removal of the solvent can be purified by crystallization from a mixture of chlorinated solvent and an ether, preferably dichloromethane and diisopropylether. The yield of the reaction is about 82%.

The Wittig reaction on compound **11** to form Latanoprost acid **12** is performed analogously to the reaction from **9a** to **10a** in the preparation of Bimatoprost.

The last step in the synthesis of **Latanoprost** is the esterification of the acid **12**, respectively, by reaction of 2-iodopropane in molar ratio with the substrate ranging from 2 to 6, more preferably about 4.5 equivalents. The reaction takes place in *N,N*-dimethylformamide in the presence of a base, preferably cesium or potassium carbonate, with molar ratio compared to the substrate from 2 to 5, preferably about 3.5.

The last two steps of the synthesis of **Travoprost,** namely the Wittig reaction from **9b** to **10b** and the esterification from **10b** to **Travoprost,** are performed analogously to what reported above for **Latanoprost** for the conversion of **11** to **12** and from **12** to **Latanoprost.**

### EXAMPLE 1

### (3aR,4S,5R,6aS)-4-[(tert-butyldimethylsilyloxy)methyl]-5-(tert-butyldiphenyl-silyloxy) hexahydro-2H-cyclopenta[b]furan-2-one

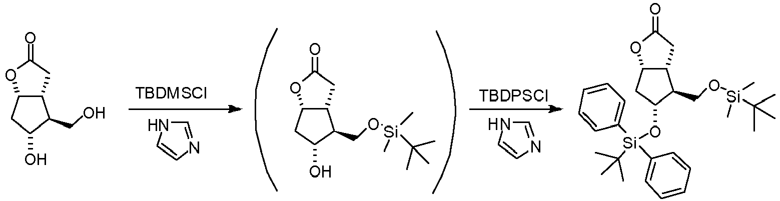

In a round bottom flask (3aR,4S,5R,6aS)-5-hydroxy-4-(hydroxymethyl)hexahydro-2H-cyclopenta[b]furan-2-one (100.0 g, 0.581 mol) was dissolved in anhydrous *N,N*-dimethylformamide (500 mL) under nitrogen atmosphere. The flask was cooled to -15°C and imidazole (42.31 g, 0.621 mol) was added followed by *tert*-butyldimethylsilyl chloride (91.91 g, 0.619 mol). The mixture was stirred at -13°C for one hour, then the temperature was allowed to reach 0°C and stirred overnight. After checking the selective protection of the primary hydroxyl group by TLC analysis, imidazole (63.26 g, 0.929 mol) and *tert*-butyl(chloro)diphenylsilane (175.2 mL, 185.19 g, 0.674 mol) were added at 0°C and the mixture was stirred at the same temperature for one hour and subsequently at room temperature. The reaction was checked by TLC and quenched by addition of ethyl acetate (1.00 L), water (1.00 L) and 5% sodium bisulfate solution (300 mL). The phases were separated and the aqueous one was extracted with ethyl acetate (330 mL). The combined organic layers were washed with water (3x 330 mL), brine (460 mL, 160 mL) and dried over magnesium sulfate. After filtration the organic solvent was removed at 40°C under reduced pressure. The crude product was obtained as a yellow oil (357.88 g) and was used in the subsequent step without further purification.

A sample (5.50 g, corresponding to 0.009 mol of starting diol) was purified by column chromatography on silica gel (eluent: *n*-hexane: ethyl acetate from 95:5 to 80:20) affording the product (4.50 g, 0.0086 mol, 95%) as a colorless oil which was used for the characterization.
**¹H-NMR** {400MHz, CDCl₃, δ (ppm)}: 7.25-7.65 (m, 10 H, Ph), 4.84 (m, 1H, CH-O-C=O), 4.09 (m, 1H, CH-O-Si), 3.40 (dd, J=5.2, 10.0Hz, 1H, CH₂-O-Si), 3.27 (dd, J=6.0, 10.0Hz, 1H, CH₂-O-Si), 2.80 (dd, J=11.2, 18.8 Hz, 1H, CH₂-C=O), 2.64 (m, 2H, -O-CH-CH₂-CH-O+ CH₂-C=O), 1.90-2.10 (m, 3H, -O-C H-CH₂-CH-O+CH+CH), 1.04 (s, 9H, (CH₃)₃C), 0.79 (s, 9H, (CH₃)₃C), 0.08 (s, 3H, (CH₃)Si), 0.07 (s, 3H, (CH₃)Si).
**¹³C-NMR** {400MHz, CDCl₃, δ (ppm)}: 177.2 (C), 135.8 (2x arom.CH), 135.7 (2x arom.CH), 133.6 (C), 133.5 (C), 129.8 (arom.CH), 129.7 (arom.CH), 127.6 (2x arom.CH), 127.5 (2x arom.CH), 84.2 (CH), 76.2 (CH), 63.2 (CH₂), 57.1 (CH), 41.0 (CH₂), 39.8 (CH), 35.8 (CH₂), 26.8 (3x CH₃), 25.7 (3x CH₃), 18.9 (C), 18.0 (C), -5.7 (2x CH₃).
**HPLC-MS** (ESI): [M-(t-Bu(CH₃)₃Si) +H₂O]⁺ = 428; [M-(t-Bu(CH₃)₃Si)+Na]⁺ = 433.

### EXAMPLE 2

### (3aR,4S,5R,6aS)-5-(tert-butyldiphenylsilyloxy)-4-(hydroxymethyl)hexahydro-2H-cyclopenta[b]furan-2-one

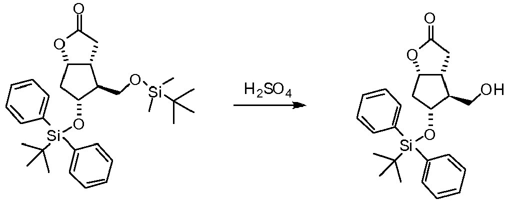

(3aR,4S,5R,6aS)-5-(*tert*-butyldiphenylsilyloxy)-4-(hydroxymethyl)hexahydro-2*H*-cyclopenta[b]furan-2-one (357.7 g, corresponding to 0.581 mmol) was dissolved in methanol (1785 mL) and aqueous sulfuric acid (63%, 14.3 mL, 21.4 g, 0.140 mmol) was added. The reaction was stirred for two hours at room temperature and 4 hours later TLC analysis revealed a complete conversion. To the reaction mixture a saturated sodium bicarbonate solution (315 mL) was added followed by water (1422 mL) and *n*-hexane (1075 mL). The solid was filtered off and washed twice with a mixture of water and *n*-hexane (2x 90 mL) at 0°C. The solid was dried under vacuum for 24 hours (222.06 g, 0.541 mol, 93% over two steps).
¹H-NMR {400 MHz, CDCl₃, δ(ppm)}: 7.5-7.6 (m, 4H, Ph), 7.2-7.33 (m, 6H, Ph), 4.70 (m, 1H, CHOC=O), 3.97 (m, 1H, CH-O-Si), 3.25 (d, J=5.2 Hz, 2H, -CH₂OH), 2.67 (dd, J=10.4, 18.0 Hz, 1H, -CH₂C=O), 2.5 (m, 1H, - CHCH₂C=O), 2.44 (dd, J=2.4, 18.0 Hz, 1H, -CH₂C=O), 1.84-2.0 (m, 3H, - CHCH₂OH + -OCHCH₂CHO-),0.93 (s, 9H, (CH₃)₃C).
¹³C-NMR {400 MHz, CDCl₃, δ(ppm)}: 135.9 (arom.CH), 177.1 (C), 135.8 (arom. CH), 133.48 (C), 133.4 (C), 129.9 (arom.CH), 129.8 (arom.CH), 127.8 (arom.CH), 127.7 (arom. CH), 83.8 (CH), 75.3 (CH), 61.9 (CH₂), 56.5 (CH), 40.7 (CH₂), 38.8 (CH), 35.5 (CH₂), 26.0 (3x CH₃), 18.9 (C).
HPLC-MS (ESI): [M+H₂O]⁺ = 428; [M+Na]⁺ = 433; [2M+Na]⁺ = 843.

### EXAMPLE 3

### (3aR,4S,5R,6aS)-5-hydroxy-4-(trityloxymethyl)hexahydro-2H-cyclopenta[b]furan-2-one

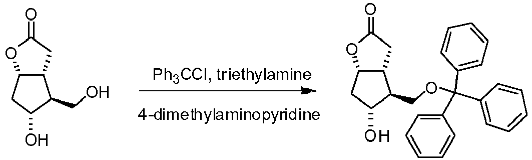

(3aR,4S,5R,6aS)-4-(hydroxymethyl)-2-oxohexahydro-2H-cyclopenta[b]furan-5-yl biphenyl-4-carboxylate (8.55 g, 0. 050 mol) was dissolved in dichloromethane (50 mL) under nitrogen atmosphere. Trityl chloride (15.2 g, 0.055 mol) was added followed by triethylamine (7.6 mL, 0.055 mol) dropwise and a solution of 4-(dimethylamino)pyridine (1.26 g, 0.010 mol) in dichloromethane (5 mL). While stirring at room temperature, the mixture turned into a red solution. The conversion of the starting material was monitored by TLC analysis for four hours. The mixture was washed with a 5% sodium bisulfate solution (40 mL) and the aqueous layer was then extracted with dichloromethane (3x 30 mL). The combined organic layers were washed with water (30 mL) and brine (30 mL). The organic layers were concentrated at 40°C under reduced pressure affording the crude product (25 g) which was suspended in *n*-hexane (100 mL) and stirred for 10 hours. After filtration the product was obtained as a pale yellow solid (24.8 g, quantitative).
**¹H-NMR** {400MHz, CDCl₃, δ (ppm)}: 7.42-7.39 (m, 5H, Ph), 7.33-7.23 (m, 10H, Ph), 4.85 (dt, J=2.8Hz, 6.8Hz, 1H, CH-O-C=O), 4.10 (q, J=6.0 Hz, 1H, CH-OH), 3.23 (dd, J=5.6, 9.6 Hz, CH₂-O-C-Ph₃), 3.12 (dd, J= 6.8, 9.6 Hz, CH₂-O-C-Ph₃), 2.70 (m, 1H, CH₂-C=O), 2.53-2.29 (m, 4H, O-CH-CH₂-CH-O+CH₂-C=O+OH+CH), 2.07-1.93 (m, 2H, O-CH-CH₂-CH-O+CH).
**¹³C-NMR** {400MHz, CDCl₃, δ (ppm)}: 35.3 (CH₂), 39.9 (CH), 40.4 (CH₂), 53.6 (CH), 64.2 (CH₂), 75.3 (CH), 83.5 (C), 87.0 (CH), 127.2 (arom.CH), 127.3 (arom.CH), 127.7 (arom.CH), 127.8 (arom.CH), 127.9 (arom.CH), 128.0 (arom.CH), 128.4 (arom. CH), 143.6 (3x arom.C), 177.2 (C).
**HPLC-MS** (ESI): [M+Na]⁺= 437; [2M+Na]⁺= 851.

### EXAMPLE 4

### (3aR,4S,5R,6aS)-5-(triphenylsilyloxy)-4-(trityloxymethyl)hexahydro-2H-cyclopenta[b]furan-2-one

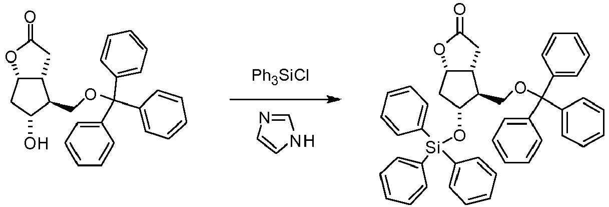

(3aR,4S,5R,6aS)-5-hydroxy-4-(trityloxymethyl)hexahydro-2H-cyclopenta[b]furan-2-one (3.6 g, 8.7 mmol) was dissolved in *N,N-*dimethylformamide (20 mL) under nitrogen atmosphere and imidazole (1.4 g, 20.6 mmol) was added followed by triphenylsilyl chloride (6.0 g, 20.3 mmol). The mixture was stirred at room temperature for 3 hours monitoring the conversion by TLC analysis. Ethyl acetate (30 mL), 5% sodium bisulfate solution (20 mL) were then added. The phases were separated and the aqueous layer was extracted with ethyl acetate (30 mL). The combined organic layers were washed with water (3x 50 mL) and brine (50 mL), filtered and concentrated under reduced pressure at 40°C. The crude product (10.0 g) was purified by column chromatography on silica gel (eluent: ethyl acetate: *n*-hexane 1:9) to obtain the title compound as a colorless solid (5.9 g, 8.7 mmol, quantitative yield).
**¹H-NMR** {400MHz, CDCl₃, δ (ppm)}: 7.56-7.21 (m, 30H, Ph), 4.77 (m, 1H, -CH-O-C=O), 4.25 (m, 1H, -CH-O-Si-), 3.06 (dd, J=6.0Hz, 9.6Hz, 1H, - CH₂-OC(Ph)₃), 2.94 (dd, J=6.8, 9.6Hz, 1H, -CH₂-OC(Ph)₃), 2.72 (dd, J=10.4, 18.4Hz, 1H, -CH₂-CO-O-), 2.62 (dd, J=3.2, 18.4Hz, 1H, -CH₂-CO-O-), 2.47 (m, 1H, -CH-CH₂-C=O), 2.26 (m, 1H, -CH-CH₂-OC(Ph)₃), 2.0 (m,1H, -CH₂-CH-O-Si-).
**HPLC-MS** (ESI): [M+Na]⁺= 695; [2M+Na]⁺= 1367.

### EXAMPLE 5

### (3aR,4S,5R,6aS)-4-(hydroxymethyl)-5-(triphenylsilyloxy)hexahydro-2H-cyclopenta[b]furan-2-one

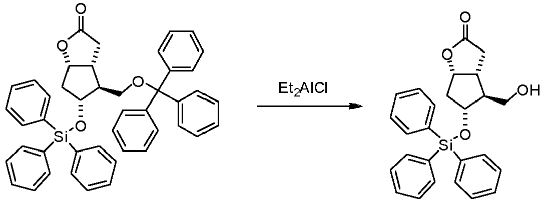

(3aR,4S,5R,6aS)-4-(hydroxymethyl)-5-(triphenylsilyloxy)hexahydro-2H-cyclopenta[b]furan-2-one (0.62 g, 0.9 mmol) was dissolved in 5.0 mL and stirred at -50°C. Diethylaluminum chloride (1.8 M, in toluene 0.5 mL) is added dropwise and the temperature is allowed to reach slowly 0°C. As the reaction did not proceed, an additional amount of diethylaluminum chloride (5 mL, 9 mmol) was added. When the reaction appeared to be complete on TLC analysis, saturated sodium bicarbonate solution was added with extreme caution. The mixture was filtered on a sintered glass filter washing the solid with ethyl acetate. The filtered phases were separated and the aqueous one was extracted with ethyl acetate (2 x 5 mL). The organic phases were concentrated under reduced pressure affording the crude product (0.40 g). After purification by column chromatography on silica gel (eluent: ethyl acetate:n-hexane = 1:1 to 6:4) the desired compound was obtained as a solid (0.20 mg, 0.40 mol, 52%).
**¹H-NMR** {400MHz, CDCl₃, δ (ppm)}: 7.65-7.63 (m, 6H, Ph), 7.48-7.39 (m, 9H, Ph), 4.84 (dt, J=2.8Hz, 6.8Hz, 1H, CH-O-C=O), 4.29 (q, J=6.0Hz, 1H, CH-O-Si-), 3.44 (d, J=4.0Hz, 2H, -CH₂-OH), 2.78 (dd, J=10.0, 17.6Hz, 1H, CH₂-C=O), 2.65, m, 1H, -O-CH-CH₂-CH-O), 2.55 (dd, J=2.4Hz, 17.6 Hz, 1H, CH₂-C=O), 2.25-2.01 (m, 3H).
**¹³C-NMR** {400MHz, CDCl₃, δ (ppm)}: 177.1 (C), 135.3 (6x arom. CH), 133.7 (3x arom.C), 130.2 (3x arom. CH), 128.0 (6x arom. CH), 83.4 (CH), 74.9 (CH), 61.6 (CH₂), 56.0 (CH), 40.8 (CH₂), 38.6 (CH), 35.2 (CH₂).
**HPLC-MS** (ESI): [M+Na]⁺= 453; [2M+Na]⁺= 883.

### EXAMPLE 6

### (3aR,4S,5R,6aS)-5-(tert-butyldiphenylsilyloxy)-4-(trityloxymethyl)hexahydro-2H-cyclopenta[b]furan-2-one

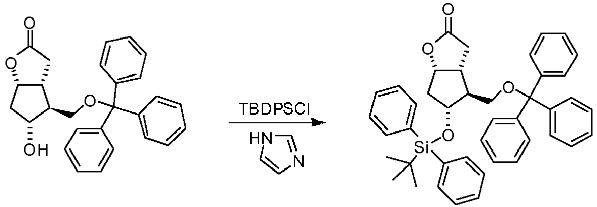

(3aR,4S,5R,6aS)-5-hydroxy-4-(trityloxymethyl)hexahydro-2H-cyclopenta[b]furan-2-one (24.8.0 g, 0.050 mol) was dissolved in *N,N-*dimethylformamide (100 mL) under nitrogen atmosphere and *tert*-butyldiphenylsilyl chloride (15.6 mL, 0.060 mol) was added followed by imidazole (4.1 g, 0.060 mol). When no more starting material was visible on TLC analysis water (150 mL) was added followed by *tert*-butylmethylether (4 mL) and a 5% sodium bisulfate solution (50 mL). The phases were separated and the aqueous layer was extracted with tert-butylmethylether (100 mL). The combined organic layers were washed with water (2x 50 mL) and brine (50 mL) and concentrated under reduced pressure at 40°C. The crude product (40.0 g, quantitative) was used in the subsequent step without further purification.

A sample was purified by column chromatography on silca gel (eluent: *n*-hexane:ethyl acetate = 9:1) and analyzed.
**¹H-NMR** {400MHz, CDCl₃, δ (ppm)}: 7.62-7.58 (m, H, Ph), 7.44-7.22 (m, 21H, Ph), 4.77 (dt, J=2.4, 7.2Hz, 1H, -CH-O-C=O), 4.06 (m, 1H, -CH-O-Si-), 3.03 (dd, J=5.8, 9.8 Hz, 1H, -CH₂-O-C(Ph)₃), 2.86 (dd, J=7.2, 9.8, 1H, - CH₂-O-C(Ph)₃), 2.79 (dd, J=10.4, 18.4 Hz, 2H, -CH₂-CO-O-), 2.53 (dd, J=3.6, 18.4 Hz, 2H, -CH₂-CO-O-), 2.50 (m, 1H, -CH-CH₂-C=O), 2.26 (m, 1H, -CH-CH₂-O-C(Ph)₃), 1.96 (ddd, J=2.4, 4.8, 14.8 Hz, 1H, -CH₂-CH-O-Si-), 1.86 (dt, J=6.8, 14.8 Hz, 1H, -CH₂-CHO-Si), 1.02 (s, 9H, C(CH₃)₃).
**HPLC-MS** (ESI): [M+Na]⁺= **675.**

### EXAMPLE 7

### (3aR,4S,5R,6aS)-5-(tert-butyldiphenylsilyloxy)-4-(hydroxymethyl)hexahydro-2H-cyclopenta[b]furan-2-one

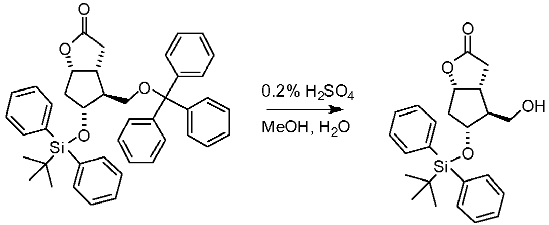

(3aR,4S,5R,6aS)-5-(*tert*-butyldiphenylsilyloxy)-4-(trityloxymethyl)hexahydro-2*H*-cyclopenta[b]furan-2-one (40.0 g, 0.050 mol) was dissolved in methanol (200 mL) at room temperature. Concentrated sulfuric acid (1.0 mL, 0.008 mol) was added dropwise and the mixture was stirred for four hours until TLC revealed that the conversion was complete. Water (200 mL) and a saturated sodium bicarbonate solution (50 mL) were added. After filtration the solid was filtered off, suspended in diisopropylether (150 mL) and stirred overnight. The product was obtained after a second filtration washing with diisopropylether (12.5 g, 0.030 mol, 61%).
**¹H-NMR** {400MHz,d₆-DMSO, δ (ppm)}: 7.43-7.41 (m, 4H, Ph), 7.29-7.21 (m, 6H, Ph), 4.72 (t, J=6.0Hz, 1H, CH-O-C=O), 4.47 (t, J=4.8 Hz, 1H, OH), 3.96 (m, 1H, CH-O-Si), 2.99 (m, 2H, CH₂-OH), 2.70 (dd, J=10.8, 18.0Hz, 1H, CH₂-C=O), 2.50 (m, 1H, CH-CH₂-C=O), 2.28 (dd, J=2.8, 18.0Hz, 1H, CH₂-C=O), 1.89 (m, 1H, CH-CH₂-OH), 1.74-1.64 (m, 2H, O-CH-CH₂-CH-O-), 0.70 (s, 9H, C(CH₃)₃).
**¹³C-NMR** {400MHz, d₆-DMSO, δ (ppm)}: 177.1 (C), 135.4 (4x arom.CH), 133.4 (C), 133.1 (C), 129.8 (2x arom.CH), 127.7 (4x arom.CH), 84.6 (CH), 76.7 (CH), 61.4 (CH₂), 57.8 (CH), 40.3 (CH₂), 39.4 (CH), 36.0 (CH₂), 26.6 (3xCH₃), 18.5, (C).
**HPLC-MS** (ESI): [M+H₂O]⁺= 428; [M+Na]⁺= 433; [2M+Na]⁺= 843.

### EXAMPLE 8

### (3aR,4R,5R,6aS)-5-(tert-butyldiphenylsilyloxy)-2-oxohexahydro-2H-cyclopenta[b]furan-4-carbaldehyde

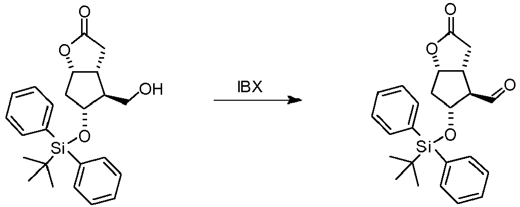

2-Iodoxybenzoic acid (240.0 g, 0.857 mol) was suspended in anhydrous dimethylsulfoxide (1200 mL) and stirred for 20 minutes at 25°C. (3aR,4S,5R,6aS)-5-(*tert*-butyldiphenylsilyloxy)-4-(hydroxymethyl)hexahydro-2H-cyclopenta[b]furan-2-one (220.0 g, 0.535 mol) was added portionwise. When TLC analysis revealed the end of the reaction after 4 hours, ethyl acetate (810 mL) and water (810 mL) were added and the mixture was filtered washing the cake in the filter twice with a mixture of ethyl acetate and water (1:1, 2 x 900 mL). The two layers of the filtrate were separated and the aqueous layer was extracted twice with ethyl acetate (2x 1000 mL). The combined organic layers were washed with water (2 x 1200 mL) and brine (1200 mL) and concentrated *in vacuo* at 40°C. An oil (240.0 g, quantitative) was obtained which was used in the following steps without further purification.
**¹H-NMR** {400 MHz, CDCl₃, δ (ppm)}: 9.35 (s, 1H, -CH=O), 7.71-7.67 (m, 4H, Ph), 7.66-7.36 (m, 6H, Ph), 4.99 (t, J=6.8Hz, 1H, -CH-O-C=O), 4.54 (m, 1H, -CH-O-Si-), 3.32 (m, 1H, -CH-CH=O), 2.91 (m, 2H, -CH₂-CO=O), 2.57 (dd, J=2.8 H, 18.4 Hz, 1H, -CH-CH₂-C=O), 2.23 (dd, J=1.2, 15.2 Hz, 1H, -CH2-CH-O-), 1.62 (m, 1H, -CH₂-CH-O), 1.07 (s, 9H, C(CH₃)₃).
**¹³C-NMR** {400 MHz, d₆-DMSO, δ (ppm)}: 198.6 (CH), 176.5 (C), 136.1 (C), 136.0 (C),135.7 (CH), 135.6 (CH), 130.1 (CH), 130.0 (CH), 129.9 (CH), 129.8 (CH), 127.8 (CH), 127.7 (CH), 127.6 (CH), 127.5 (CH), 84.6 (CH), 74.5 (CH), 68.0 (CH), 40.4 (CH₂), 36.2 (CH), 35.7 (CH₂), 26.7 (3xCH₃), 18.8 (C).

### EXAMPLE 9

### (3aR,4R,5R,6aS)-5-(tert-butyldiphenylsilyloxy)-4-((E)-3-oxo-5-phenylpent-1-enyl)hexahydro-2H-cyclopenta[b]furan-2-one

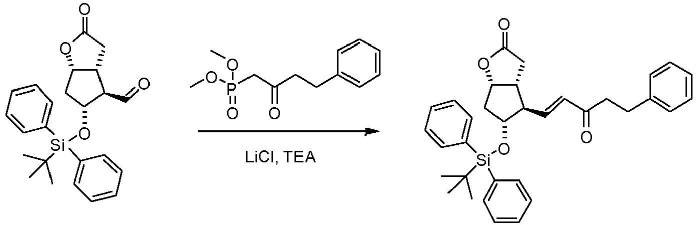

Dimethyl 2-oxo-4-phenylbutylphosphonate (244.65 g, 0.955 mol) was suspended in tetrahydrofuran (1600 mL) under nitrogen atmosphere. The mixture was cooled to -10°C; lithium chloride (40.47 g, 0.954 mol) and triethylamine (132.5 mL, 96.7 g, 0.955 mol) were added and the mixture was stirred at -10°C for 1.5 hour. Subsequently a solution of (3aR,4R,5R,6aS)-5-(*tert*-butyldiphenylsilyloxy)-2-oxohexahydro-2H-cyclopenta[b] furan-4-carbaldehyde (142.5 g, 0.350 mol) in tetrahydrofuran (400 mL) was added dropwise over 25 minutes. The system was stirred at -10°C and the reaction appeared to be complete on TLC analysis after 2 hours.

Ethyl acetate (850 mL), water (850 mL) and saturated sodium bicarbonate solution (23 mL) were added. After separation of the layers, the aqueous one was extracted with ethyl acetate (850 mL) and the combined organic layers were washed with 0.3 M sulfuric acid solution (1030 mL) and brine (1100 mL). The removal of the organic solvent under reduced pressure afforded the crude product (368.81 g). Purification was made by column chromatography (eluent: *n*-hexane:ethyl acetate = 2:1); after combining the fractions of interest and removing the solvents under reduced pressure, the title compound was obtained as an oil (137.0 g, 0.254 mol, 72%).
¹H-NMR {400 MHz, CDCl₃, δ (ppm)}: 7.53-7.64 (m, 4H, Ph), 7.13-7.47 (m, 11H, Ph), 6.31 (dd, J=7.6, 15.6 Hz, 1H, vinyl), 5.95 (d, J= 15.6 Hz, 1H, vinyl), 4.82 (m, 1H, CH-OH), 3.98 (m, 2H, CH-O-C=O + CH-O-Si), 2.03-2.91 (m, 10H), 1.03 (s, 9H, (CH₃)₃C-).
¹³C-NMR {400 MHz, CDCl₃, δ (ppm)}: 198.4 (C), 176.1 (C), 144.4 (CH), 140.9 (C), 135.8 (CH), 133.1 (C), 132.9 (C), 131.2 (CH), 129.9 (CH), 128.4 (CH), 128.3 (CH), 127.7 (CH), 126.1 (CH), 82.7 (CH), 78.1 (CH), 56.7 (CH), 42.2 (CH₂), 41.7 (CH), 40.6 (CH₂), 34.5 (CH₂), 29.8 (CH₂), 26.7 (3x CH₃), 18.9 (C).
HPLC-MS (ESI): [M+H]⁺ = 539; [M+H₂O]⁺ = 556; [2M+Na]⁺ = 1099.

### EXAMPLE 10

### (3aR,4R,5R,6aS)-5-(tert-butyldiphenylsilyloxy)-4-((S,E)-3-hydroxy-5-phenylpent-1-enyl)hexahydro-2H-cyclopenta[b]furan-2-one

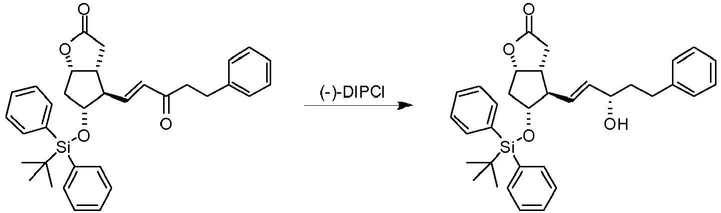

(3aR,4R,5R,6aS)-5-(*tert*-butyldiphenylsilyloxy)-4-((E)-3-oxo-5-phenylpent-1-enyl)hexahydro-2*H*-cyclopenta[b]furan-2-one (48.60 g, 0.090 mol) was dissolved in tetrahydrofuran (180.0 mL) under nitrogen atmosphere and cooled to 0°C. A 60% solution of (-)-β-chlorodiisopinocampheylborane in tetrahydrofuran (120.6 g, 0.226 mol) was added dropwise and the reaction was checked by TLC analysis. The conversion was complete after 2 hours; methanol (12 mL) was added followed by water (240 mL) and a saturated sodium bisulfate solution (72 mL). The two layers were separated and the inorganic one was extracted with ethyl acetate (2x 240 mL). The combined organic layers were washed with brine (250 mL) and concentrated at 40°C under reduced pressure affording the crude product as an oil (120.5 g). A first purification on silica gel was performed (eluting first with diisopropyl ether, then with ethyl acetate) affording 52.3 g of pale yellow oil.

A second chromatografic purification on silca gel (eluent heptane:ethyl acetate = 9:1, gradually increasing the percentage of ethyl acetate) allowed to isolate the product with an amount of the undesired (S)-epimer lower than 0.5% (34.0 g, 0.063 mol, 70%).
¹H-NMR {400 MHz, CDCl₃, δ (ppm)}: 7.63-7.68 (m, 4H, Ph), 7.36-7.47 (m, 6H, Ph), (7.15-7.31 (m, 5H, Ph), 5.42 (dd, J=6.4, 15.4Hz, 1H, vinyl), 5.20 (dd, J=8.0, 15.4Hz, 1H, vinyl), 4.82 (m, 1H, CH-OH), 3.97 (m, 2H, CHO-C=O+CH-O-Si), 2.43-2.76 (6H, m), 2.01-2.18 (m, 2H), 1.66-1.82 (m, 2H), 1.06 (s, 9H, (CH₃)₃C).
¹³C-NMR {400 MHz, CDCl₃, δ (ppm)}: 176.8 (C), 141.6 (C), 135.9 (CH), 135.3 (CH), 133.5 (C), 133.3 (C), 129.9 (CH), 129.8 (CH), 129.7 (CH), 128.3 (CH), 127.6 (CH), 127.5 (CH), 125.8 (CH), 83.0 (CH), 78.4 (CH), 71.4 (CH), 56.6 (CH), 42.0 (CH), 40.3 (CH₂), 38.5 (CH₂), 34.5 (CH₂), 31.6 (CH₂), 26.7 (3x CH₃), 19.0 (C).
HPLC-MS (ESI): [M+H₂O]⁺= 558; [M-Ph]⁺ = 463.

### EXAMPLE 11

### (3aR,4R,5R,6aS)-5-(tert-butyldiphenylsilyloxy)-4-((S,E)-3-hydroxy-5-phenylpent-1-enyl)hexahydro-2H-cyclopenta[b]furan-2-ol

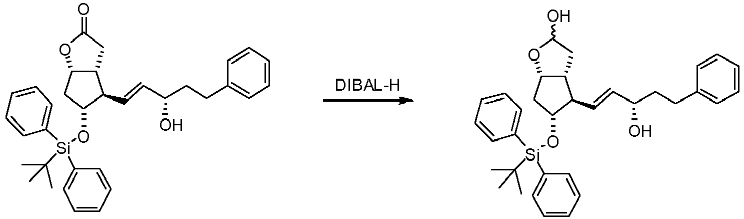

(3aR,4R,5R,6aS)-5-(*tert*-butyldiphenylsilyloxy)-4-((R)-3-hydroxy-5-phenylpentyl) hexahydro-2*H*-cyclopenta[b]furan-2-one (10.0 g, 0.019 mol) was dissolved in anhydrous tetrahydrofuran (100.0 mL) at room temperature under nitrogen atmosphere. The solution was cooled to -78°C and a solution of diisobutylaluminum hydride in toluene (1.7 M, 45.0 mL, 0.077 mmol) was added dropwise over 30 minutes. After checking the conversion by TLC analysis the reaction appeared to be complete after three hours and methanol (100 mL) was added dropwise. The mixture was allowed to reach room temperature. After filtering the solid and washing the filter with a solution of dichloromethane (100 mL) and methanol (25 mL), the filtrate was concentrated under reduced pressure at 40°C. The crude product was obtained as an oil (7.91 g, 0.015 mol). The solid was suspended in water (200 mL) and hydrochloric acid (1.6 M, 100 mL) and extracted with ethyl acetate (200 mL). The phases were separated and the solvent of the organic layer was removed under vacuum affording an additional portion of product (1.28 g, 0.002 mol, 89%).
¹H-NMR {400 MHz, CDCl₃, δ (ppm)}: 7.73-7.66 (m, 4H, Ph); 7.46-7.17 (m, 11H, Ph), 5.68 (m, 0.7H, CHOH, major-isomer); 5.56 (t, J= 4.8Hz, 0.3H, CHOH, minor-isomer); 5.53-5.44 (m, 1H, vinyl); 5.29-5.20 (m, 1H, vinyl); 4.50 (m, 0.7H, -C=C-CH-OH, major-isomer); 4.40 (m, 0.3H, -C=C-CH-OH, minor-isomer); 3.93 (m, 2H, -CH-O-CH-OH+-CH-O-Si); 3.48 (m, 1H, OH); 2.64 (m, 2H, -CH₂Ph); 1.08 (s, 9H, (CH)₃C); 2.38-1.27 (m, 9H).
¹³C-NMR {400 MHz, CDCl₃, δ (ppm)}: 141.7 (141.8) (C), 135.9 (136.0) (CH), 133.9 (134.4) (C), 133.7 (134.2) (C), 131.8 (132.4) (CH), 129.7 (2xCH, Ph), 129.6 (2xCH, Ph), 128.3 (2xCH, Ph), 127.5 (2xCH, Ph), 125.8 (CH, Ph), 98.8 (100.8) (CH), 80.1 (82.3) (CH), 79.0 (79.1) (CH), 71.9 (72.0) (CH), 56.3 (56.7) (CH), 44.7 (45.4) (CH), 40.5 (43.1) (CH₂), 38.9 (CH₂), 38.5 (CH₂), 31.7 (CH₂), 28.6 (3xCH₃).
HPLC-MS (ESI): [M+Na]⁺ = 565.

### EXAMPLE 12

### (3aR,4R,5R,6aS)-4-((S,E)-3-hydroxy-5-phenylpent-1-enyl)hexahydro-2H-cyclopenta[b]furan-2,5-diol

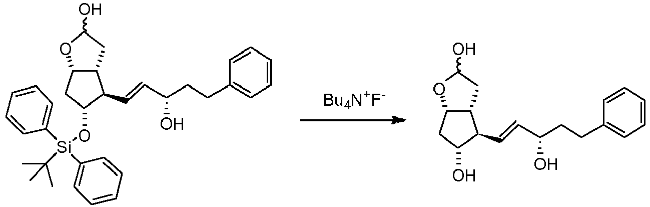

(3aR,4R,5R,6aS)-5-(*tert*-butyldiphenylsilyloxy)-4-((R)-3-hydroxy-5-phenylpentyl) hexahydro-2*H*-cyclopenta[b]furan-2-ol (7.90 g, 0.015 mol) was dissolved in tetrahydrofuran (100 mL) and cooled to 0°C. Tetrabutylammonium fluoride trihydrate (19.0 g, 0.060 mol) was slowly added. The reaction was stirred at 0°C for 4 hours and checked by TLC analysis. Maintaining the temperature at 0°C, water (100 mL) and ethyl acetate (100 mL) were added and, after stirring, the layers were separated. The aqueous phase was extracted with additional ethyl acetate (2x 100 mL) and the combined organic layers were washed with brine (100 mL). After removing the organic solvents a yellow oil was obtained (8.19 g).

The product was purified by column chromatography on silica gel (eluent dichloromethane:methanol=94:6) affording a yellow-orange oil containing two isomers (α-lactol and β-lactol) (4.10 g, 0.013 mol, 89%).
¹H-NMR {400 MHz, CDCl₃, δ (ppm)}: 7.27-7.12 (m, 5H, Ph); 5.63-5-42 (m, 3H, -CH=CH- + -O-CH-OH); 4.59 (dt, J=3.6, 6.8 Hz, 1H, -C=C-CH-OH, major isomer); 4.55 (dt, J=4.0, 7.6 Hz, 1H, -C=C-CH-OH minor isomer); 4.06 (m, 1H, -CH-O-CHOH); 3.84 (m, 1H, -CH-OH), 2.72-2.62 (m, 2H, -CH₂-Ph), 2.42-1.76 (m, 8H, other hydrogens).
¹³C-NMR {400 MHz, CDCl₃, δ (ppm)}: 141.7 (141.8)(C), 134.7 (135.2) (CH), 132.2 (133.1) (CH), 128.4 (2xCH, Ph), 128.3 (2xCH, Ph), 125.8 (CH arom.), 97.7 (100.7) (CH), 80.3 (83.2) (CH), 77.4 (78.0) (CH), 72.2 (72.3) (CH), 56.7 (57.1) (CH), 45.6 (46.3) (CH), 42.5 (CH₂), 38.9 (39.7) (CH₂), 38.6 (CH₂), 31.7 (31.8) (CH₂).
HPLC-MS (ESI): [M+Na]⁺ = 327, [M+K]⁺ = 343.

### EXAMPLE 13

### (Z)-7-((1R,2R,3R,5S)-3,5-dihydroxy-2-((S,E)-3-hydroxy-5-phenylpent-1-enyl)cyclopentyl)hept-5-enoic acid (Bimatoprost free acid)

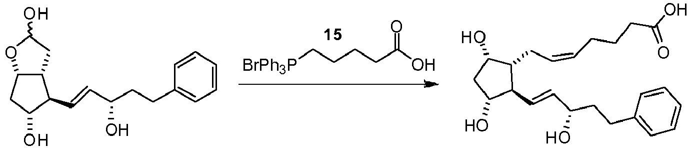

4-Carboxybutyltriphenylphosphonium bromide **15** (65.4 g, 0.148 mol) was suspended in tetrahydrofuran (150.0 mL) at 0°C under nitrogen atmosphere. A solution of potassium *tert*-butoxide in tetrahydrofuran (296.0 mL, 0.296 mol) was added dropwise and the mixture turned into orange. After stirring for 45 minutes at 0°C the system was cooled to -15°C. A solution of (3aR,4R,5R,6aS)-4-((S,E)-3-hydroxy-5-phenylpent-1-enyl)hexahydro-2H-cyclopenta[b]furan-2,5-diol (10.0 g, 0.033 mol) in tetrahydrofuran (46.0 mL) was added dropwise at a temperature lower than - 10°C. After three hours at -15°C no more starting material was visible on TLC and water (200 mL) was added. The mixture was extracted with diisopropyl ether (144 mL) and the layers were separated. The aqueous phase was treated with 0.6 N HCl to pH 6.0. Two extractions with ethyl acetate (2x 250 mL) were then performed and the combined organic layers were concentrated under vacuum at 40°C. An oil (26.80 g) was obtained which was purified on silica gel (eluent: dichloromethane:methanol from 97.5:2.5 to 85:15). The fractions of interest were combined and concentrated at 35°C under reduced pressure affording a colorless oil (11.7 g, 0.030 mol, 91%).
¹H-NMR {400 MHz, CDCl₃, δ (ppm)}: 7.29-7.25 (m, 2H, Ph), 7.19-7.15 (m, 3H, Ph), 5.60 (dd, J=7.2, 15.2 Hz, 1H, vinyl), 5.51-5.41 (m, 2H, H vinyl), 5.38-5.31 (m, 1H, vinyl), 4.5-3.8 (m, 7H), 2.67 (m, 2H, -CH₂-Ph), 2.37-1.43 (m, 14H).
¹³C-NMR {400 MHz, CDCl₃, δ (ppm)}: 177.2 (C), 141.9 (C), 134.9 (CH), 133.0 (CH), 129.6 (CH), 129.1 (CH), 128.4 (2xCH, arom.), 128.3 (2xCH arom.),125.8 (CH), 77.4 (CH), 72.3 (CH), 72.2 (CH), 55.1 (CH), 50.5 (CH), 42.7 (CH₂), 38.5 (CH₂), 32.9 (CH₂), 31.8 (CH₂), 26.3 (CH₂), 25.2 (CH₂), 24.4 (CH₂).
HPLC-MS (ESI): [M-H₂O+1]⁺= 371; [M+Na]⁺ = 411; [2M+Na]⁺ = 799.

### EXAMPLE 14

### (5Z)-7-[(2R)-3,5-Dihydroxy-2-[(1E)-3-hydroxy-5-phenylpent-1-en-1-yl]cyclopentyl]-N-ethylhept-5-enamide (Bimatoprost)

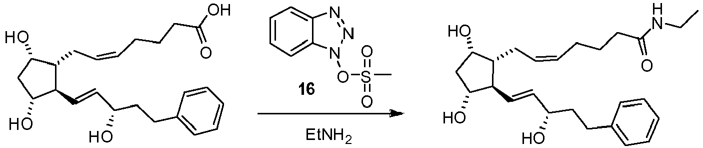

Bimatoprost acid (11.50 g, 0.030 mol) was dissolved in dimethylformamide (92.0 mL), and stirred at -15°C. Triethylamine (7.25 mL, 5.26 g, 0.052 mol) was then added over 5 minutes followed by the portionwise addition of 1-(methylsulfonyloxy)benzotriazole **16** (prepared according to Bulletin of the Chemical Society of Japan, 1978, 51(11), 3320-3329) (11.27 g, 0.053 mol). The mixture was then stirred for one hour at -15°C and an aqueous solution of ethylamine (70% weight, 8.4 mL, 0.104 mol) was added dropwise over 5 minutes. The temperature was allowed to reach 0°C and the reaction was checked by TLC. The mixture was washed with water (172.0 mL) and extracted four times with ethyl acetate (4x 230.0 mL). The combined organic layers were washed with 5% sodium bisulfate solution (100 mL, 50 mL, 50 mL). The bisulfate aqueous phases were extracted with ethyl acetate (50.0 mL). The organic layers were concentrated at 40°C under reduced pressure affording the crude product as an oil (16.98 g). Treatment with dichloromethane and diisopropylether at 0°C for one hour followed by filtration afforded a solid which was then recrystallized from ethyl acetate (6.79 g, 0.016 mol, 55%).
¹H-NMR {400 MHz, CDCl₃, δ (ppm)}: 7.26 (m, 2H, Ph), 7.17 (m, 3H, Ph), 6.07 (t, J=5.6Hz, 1H, -NH-), 5.57 (dd, J=7.6, 15.2 Hz, 1H, H-14), 5.45 (dd, J=8.8, 15.2 Hz, 1H, H-13), 5.35 (m, 2H, H-5+H-6), 4.32 (d, J=4.8 Hz, 1H, OH-11), 4.08 (m, 2H, H-9+H-15), 3.90 (m, 1H, H-11), 3.73 (m, 2H, OH-9+OH-15), 3.20 (m, 2H, -N-CH₂-CH₃), 2.64 (m, 2H, -CH₂-17), 2.30 (m, 1H, H-12), 2.18 (m, 2H, H-7+H-10), 2.11 (m, 3H, CH₂-2+H-7), 2.03 (m, 2H, H-4), 1.89 (m, 1H, H-16), 1.77 (m, 2H, H-10+H-16), 1.64 (m, 2H, H-3), 1.45 (m, 1H, H-8), 1.09 (t, J=6.8Hz, 3H, -N-CH₂-CH3).
¹³C-NMR {400 MHz, CDCl₃, δ (ppm)}: 173.4 (C), 142.0 (C), 135.0 (CH), 133.2 (CH), 129.6 (CH), 129.1 (CH), 128.4 (2xCH arom), 128.3 (2xCH arom), 125.7 (CH arom), 77.5 (CH), 72.22 (CH), 72.20 (CH), 55.4 (CH), 50.1 (CH), 42.8 (CH₂), 38.7 (CH₂), 35.8 (CH₂), 34.3 (-N-CH₂), 31.8 (CH₂), 26.6 (CH₂), 25.6 (CH₂), 25.3 (CH₂), 14.7 (CH₃).
HPLC-MS (ESI): [M+Na]⁺ = 438, [(M-H₂O) +H]⁺=398, [(M-2H₂O) +H]⁺=380.

### EXAMPLE 15

### (3aR,4R,5R,6aS)-4-((R)-3-hydroxy-5-phenylpentyl)hexahydro-2H-cyclopenta[b]furan-2,5-diol

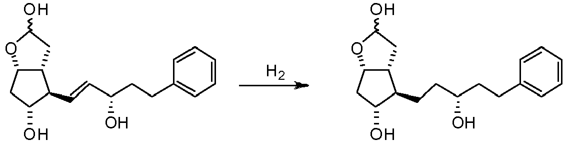

A suspension of 5% platinum on carbon (8.0 g) in methanol (60 mL) was saturated with hydrogen stirring for two hours at room temperature. A solution of (3aR,4R,5R,6aS)-4-((S,E)-3-hydroxy-5-phenylpent-l-enyl)hexahydro-2H-cyclopenta[b]

furan-2,5-diol (20.0 g, 0.066 mol) and triethylamine (18 mL, 0.129 mol) in methanol (40 mL) was added over 15 minutes and stirred for 24 hour. The catalyst was subsequently filtered off and washed with methanol. The solvent was removed at 35°C under reduced pressure affording the crude product as an oil (24.5 g).

A sample of the oil (5 g) was dissolved in dichloromethane/diisopropyl ether and cooled until a solid appeared. After stirring for one hour the solid was filtered off and the product was obtained as a white solid (16.6 g, 0.054 mol, 82%).
¹H-NMR {400 MHz, CDCl₃, δ (ppm)}: 7.28-7.13 (m, 5H, Ph); 6.11 (d, J=4.0Hz, OH acetal minor isomer), 5.80 (d, J=4.0Hz, OH acetal major isomer); 5.39 (t, J=3.6Hz, H, acetal major isomer), 5.32 (t, J=3.6Hz, H, acetal minor isomer), 4.77 (d, J=6.0 Hz, OH minor isomer), 4.62 (d, J=5.6Hz, OH major isomer), 4.40 (d, J=5.6 Hz, 1H, OH), 4.34 (dt, J=6.0Hz, 6.8 Hz, -C-O-CH-CH₂-, major isomer), 4.27 (m, -CO-CH-CH₂-, minor isomer), 3.62 (m, 1H, -CH₂-CH-OH), 3.38 (m, 1H, -CH₂-CH-OH), 2.73-2.55 (m, 2H, -CH₂-Ph), 2.17-1.05 (m, 12H, other protons).
¹³C-NMR {400 MHz, CDCl₃, δ (ppm)}: 142.6 (C), 128.3 (2xCH, arom), 128.2 (2xCH, arom), 125.5 (CH arom.), 99.1 (99.8) (CH), 78.8 (80.8) (CH), 77.1 (76.8) (CH), 69.5 (67.1) (CH), 53.3 (52.2) (CH), 44.9 (45.6) (CH), 41.0 (42.9) (CH₂), 40.9 (40.8) (CH₂), 39.0 (CH₂), 35.4 (CH₂), 31.6 (CH₂), 28.7 (CH₂).
HPLC-MS (ESI): [M+Na]⁺ = 329, [2(M-OH)+H]⁺=577.

### EXAMPLE 16

### (Z)-7-((1R,2R,3R,5S)-3,5-dihydroxy-2-((R)-3-hydroxy-5-phenylpentyl)cyclopentyl)hept-5-enoic acid (Latanoprost acid)

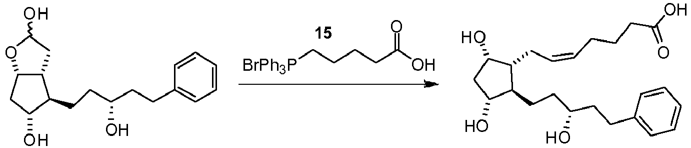

4-Carboxybutyltriphenylphosphonium bromide **15** (32.7 g, 0.074 mol) was suspended in tetrahydrofuran (75.0 mL) at 0°C under nitrogen atmosphere. A 1M solution of potassium *tert*-butoxide in tetrahydrofuran (296.0 mL, 0.296 mol) was added dropwise and the mixture turned into orange. After stirring for 45 minutes at 0°C the system was cooled to -15°C. A solution of (3aR,4R,5R,6aS)-4-((R)-3-hydroxy-5-phenylpentyl)hexahydro-2H-cyclopenta[b]furan-2,5-diol (5.0 g, 0.016 mol) in tetrahydrofuran (23.0 mL) was added dropwise at a temperature lower than -10°C. After stirring overnight at -15°C no more starting was visible on TLC and water (100 mL) was added. The mixture was extracted with diisopropyl ether (70 mL) and after separation the aqueous phase was treated with 0.6 N HCl to pH 6.0. Three extractions with ethyl acetate (3x 125 mL) were then performed, each time adjusting the pH of the aqueous phase to 6.0. The combined organic layers were concentrated under vacuum at 35°C. An oil (13.87 g) was obtained which was used in the subsequent step without further purification.
¹H-NMR {400 MHz, CDCl₃, δ (ppm)}: 7.71 (m, 1H, Ph), 7.49 (m, 1H, Ph), 7.30-7.17 (m, 3H, Ph), 5.52-5.35 (m, 2H, -CH=CH-), 4.34 (bs, 4H, OH), 4.17 (m, 1H, -CH-OH(C-9)), 3.96 (m, 1H, -CH-OH (C-11)), 2.78 (m, 1H, -CH-OH (C-15)), 2.78 (m, 1H, -CH₂Ph), 2.66 (m, 1H, -CH₂Ph), 2.36-1.27 (m, 18H).
¹³C-NMR {400 MHz, CDCl₃, δ (ppm)}: 176.5 (C), 142.2 (C), 130.8 (CH), 130.7 (CH), 129.4 (CH), 128.8 (CH), 128.7 (CH), 128.4 (CH), 125.7 (CH), 78.3 (CH), 74.2 (CH), 71.4 (CH), 52.2 (CH), 51.6 (CH), 42.4 (CH₂), 38.8 (CH₂), 35.2 (CH₂), 33.4 (CH₂), 32.0 (CH₂), 29.1 (CH₂), 26.6 (CH₂), 26.4 (CH₂), 24.7 (CH₂).
HPLC-MS (ESI): [M+Na]⁺ = 413, [M+H]⁺ = 391.

### EXAMPLE 17

### (Z)-isopropyl 7-((1R,2R,3R,5S)-3,5-dihydroxy-2-((R)-3-hydroxy-5-phenylpentyl)cyclopentyl)hept-5-enoate (Latanoprost)

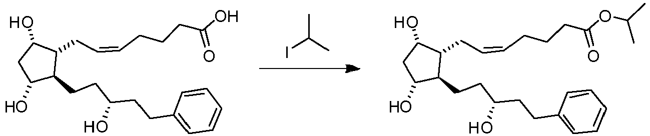

Latanoprost acid (6.78 g, corresponding to 0.008 mol) was dissolved in *N*,*N*-dimethylformamide (108 mL) and cesium carbonate (8.48 g, 0.026 mol) was added at room temperature. 2-Iodopropane (3.46 mL, 0.035 mol) was added and the suspension was stirred at 40°C for 3 hours, checking the conversion on TLC. The mixture was then allowed to reach 25°C and a mixture of ice (184 g), water (40 mL), sodium thiosulfate (1M, 18 mL), was added stirring at -5/0°C for 15 minutes. The mixture was extracted with *tert*-butylmethylether (285 mL) and the phases were separated. The aqueous phase was extracted twice with *tert*-butylmethyl ether (2x 200 mL) and the combined organic layers were washed with brine (176 mL, 130 mL). The organic phase was concentrated under reduced pressure at 25°C and the crude product was obtained as a yellow oil (6.60 g). Purification by column chromatography on silica gel was performed eluting with dichloromethane:methanol increasing the percentage of methanol from 0 to 5%. A second purification on silica gel afforded Latanoprost (2.36 g, 0.005 mol, 68% over two steps).
¹H-NMR {400 MHz, CDCl₃, δ (ppm)}: 7.32-7.19 (m, 5H, Ph), 5.45-5.51 (m, 2H, H-5 e H-6 vinyl), 5.0 (hept, J=6.3 Hz, 1H, CH₃CHCH₃), 4,18 (bs, 1H, CHOH), 3.95 (bs, 1H, CHOH), 3.67 (bs, 1H, CHOH), 2.76 (m, 2H, CH₂Ph), 1.23 (d, J=6.3Hz, 6H, C(CH₃)₂), 2.55-1.3, 21H).
HPLC-MS (ESI): [M+Na]⁺ = 455, [M+H]⁺ = 432.

## Claims

1. Process for the preparation of PGF_{2α} derivatives of formula (I) 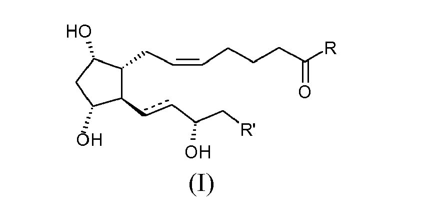 wherein:
1) R is (CH₃)₂CHO-, R' is benzyl and ---- in formula (I) is a single bond,
2) R is (CH₃)₂CHO-, R' is (3-trifluoromethyl)benzyloxy and ---- in formula (I) is either a single or a double bond
3) R is CH₃CH₂NH-, R' is benzyl and ---- in formula (I) is either a single or a double bond,
according to the following reaction scheme: 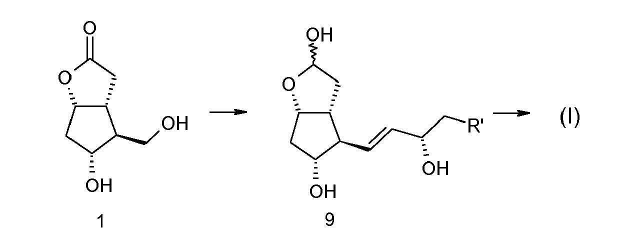 **characterized in that** compound **1** is converted into compound **9** through the following steps: 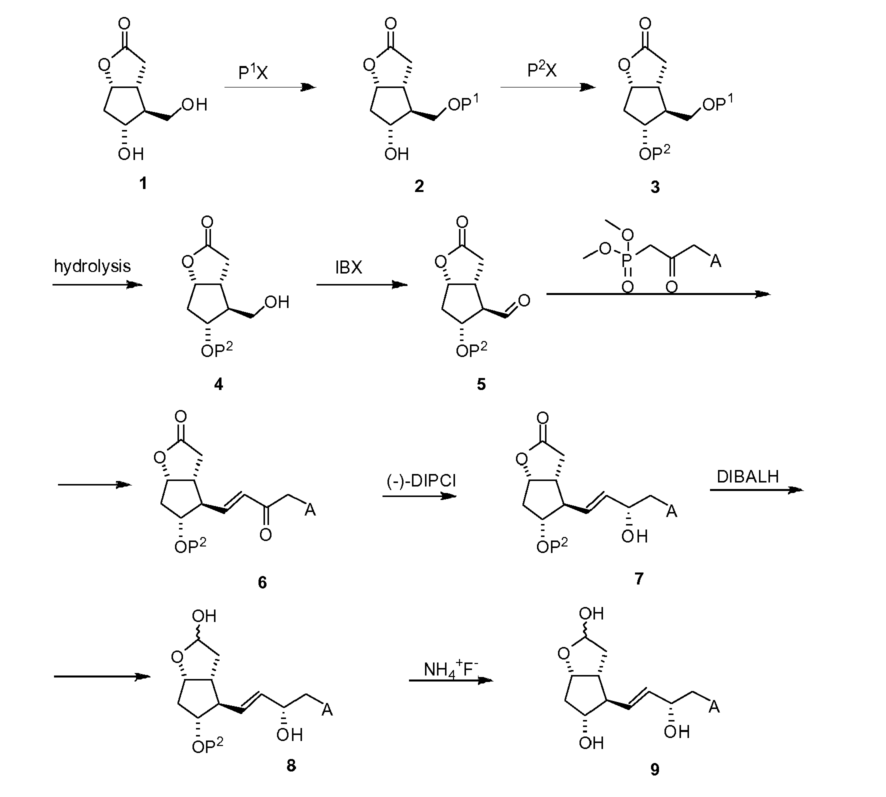 wherein
P¹ = triarylmethyl optionally substituted on the aryl moiety; -SiQ¹₃ wherein Q¹ independently from one another represents (C₁-C₄)alkyl, (C₆-C₁₀)aryl, (C₁-C₄)alkyl(C₆-C₁₀)aryl or (C₁-C₄)alkoxy(C₆-C₁₀)aryl
P² = -SiQ²₂Q³ wherein Q² = (C₆-C₁₀)aryl and Q³ = (C₁-C₄)alkyl with the condition that P¹≠P²
or P¹ and P² are selected from the following pairs:
P¹ = *tert*-butyldimethylsilyl- and P² = *tert*-butyldiphenylsilyl-
P¹ = triphenylmethyl- and P² = *tert*-butyldiphenylsilyl-
P¹ = triphenylmethyl- and P² = triphenylsilyl-
X = halogen, -OSO₂CF₃
A = benzyl or (3-trifluoromethyl)benzyloxy
(-)-DIPCl =(-)-diisopinocampheylborane
DIBALH= diisobutylaluminium hydride
IBX= 2-iodoxybenzoic acid.

2. Process according to claim 1, wherein the conversion of compound **1** into compound **3** with P¹= *tert*-butyldimethylsilyl- and P²= *tert*-butyldiphenylsilyl- is a one-pot process carried out in *N*,*N*-dimethylformamide as a solvent by reaction first with *tert-*butyldimethylsilyl chloride and imidazole at a temperature between -15 and 5°C until complete conversion to **2**, followed by direct addition into the reaction mixture of a second portion of imidazole and *tert-*butyldiphenylsilylchloride at a temperature between 0°C and 25°C.

3. Process according to claim 1, wherein the oxidation of compound **4** to compound **5** is carried out with IBX at a temperature ranging from 0°C to 40°C with a molar ratio between IBX and compound **4** ranging from 1 to 4 in a solvent selected from dimethyl sulfoxide, *N*,*N*-dimethylformamide, tetrahydrofuran, methyltetrahydrofuran or a mixture thereof.

4. Process according to claims 1-3, wherein compound **9a**, corresponding to compound **9** in which A=benzyl, is converted into Bimatoprost, corresponding to compound (I) wherein R=CH₃CH₂NH-, R'= benzyl and ---- is a double bond, according to the following steps:

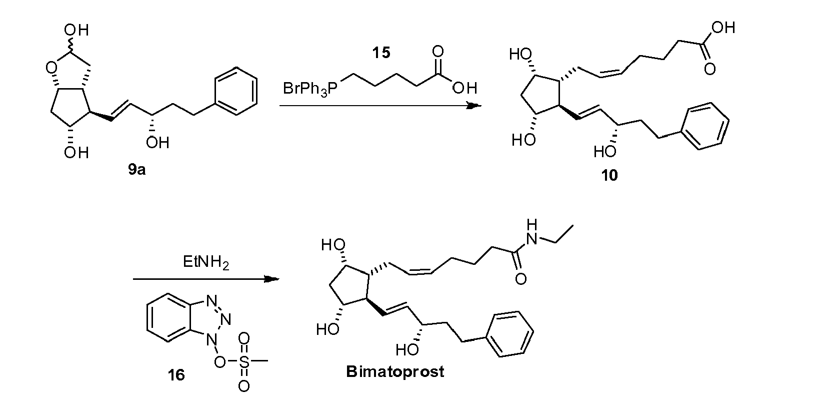

5. Process according to claim 4, wherein the conversion of compound 10 to Bimatoprost is performed by reaction with ethylamine in an organic solvent at a temperature between -35 and 25°C in the presence of triethylamine and a suitable coupling reagent.

6. Process according to claim 5, wherein the molar ratio between compound **10** and ethylamine is comprised between 1 and 5.

7. Process according to claim 6, wherein said molar ratio is 3.5.

8. Process according to claim 5, wherein the coupling reagent is 1-(methylsulfonyloxy)benzotriazole.

9. Process according to claim 5, wherein the molar ratio between compound **10** and the coupling reagent is comprised between 1 and 2.5.

10. Process according to claim 9, wherein said molar ratio is 1.8.

11. Process according to claim 5, wherein the organic solvent is chosen among amides, ethers, ketones and chlorinated solvents.

12. Process according to claim 11, wherein the organic solvent is *N,N-*dimethylformamide.

13. Process according to claim 5, wherein the reaction is performed at a temperature comprised between -20°C and -10°C in the presence of triethylamine.

14. Process according to claims 1-3, wherein compound 9a, corresponding to compound **9** in which A=benzyl, is converted into latanoprost, corresponding to compound (**I**) wherein R=(CH₃)₂CHO-, R'= benzyl and ---- is a single bond, according to the following steps:

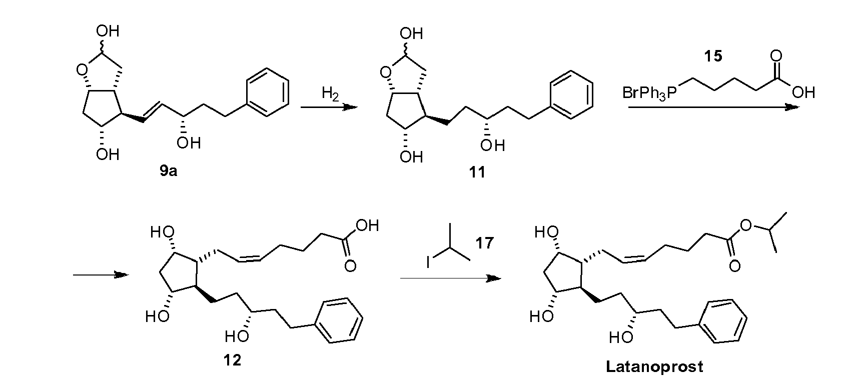

15. Process according to claims 1-3, wherein compound **9b**, corresponding to compound **9** in which A=(3-trifluoromethyl)benzyloxy, is converted into travoprost, corresponding to compound (**I**) wherein R=(CH₃)₂CHO-, R'= (3-trifluoromethyl)benzyloxy and ---- is a double bond, according to the following steps:

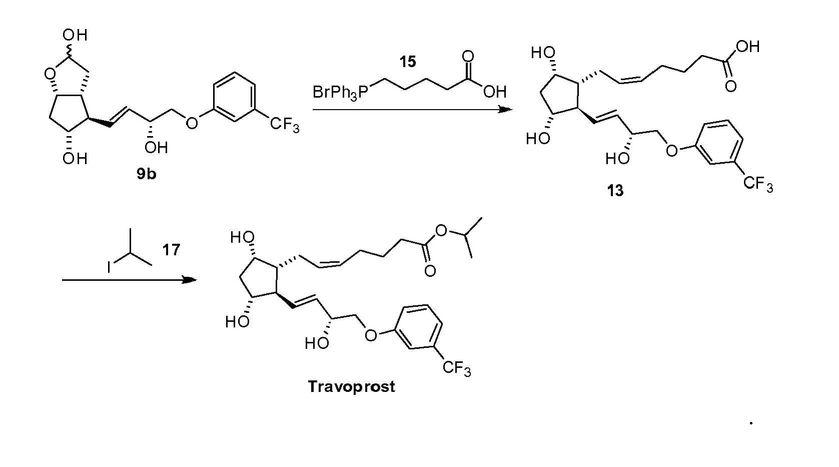

## Patentansprüche

1. Verfahren zur Herstellung von PGF_{2α}-Derivaten der Formel (I) 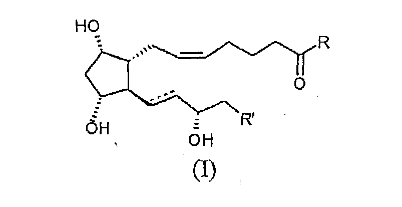 worin:
1) R (CH₃)₂CHO- ist, R' Benzyl ist und ---- in Formel (I) eine Einfachbindung ist,
2) R (CH₃)₂CHO- ist, R' (3-Trifluormethyl)benzyloxy ist und ---- in Formel (I) entweder eine Einfach- oder eine Doppelbindung ist,
3) R CH₃CH₂NH- ist, R' Benzyl ist und ---- in Formel (I) entweder eine Einfach- oder eine Doppelbindung ist, gemäß dem nachfolgenden Reaktionsschema: 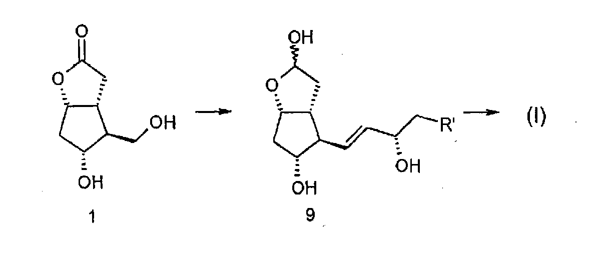
**dadurch gekennzeichnet, dass** Verbindung 1 überführt wird in Verbindung 9 durch die nachfolgenden Schritte: 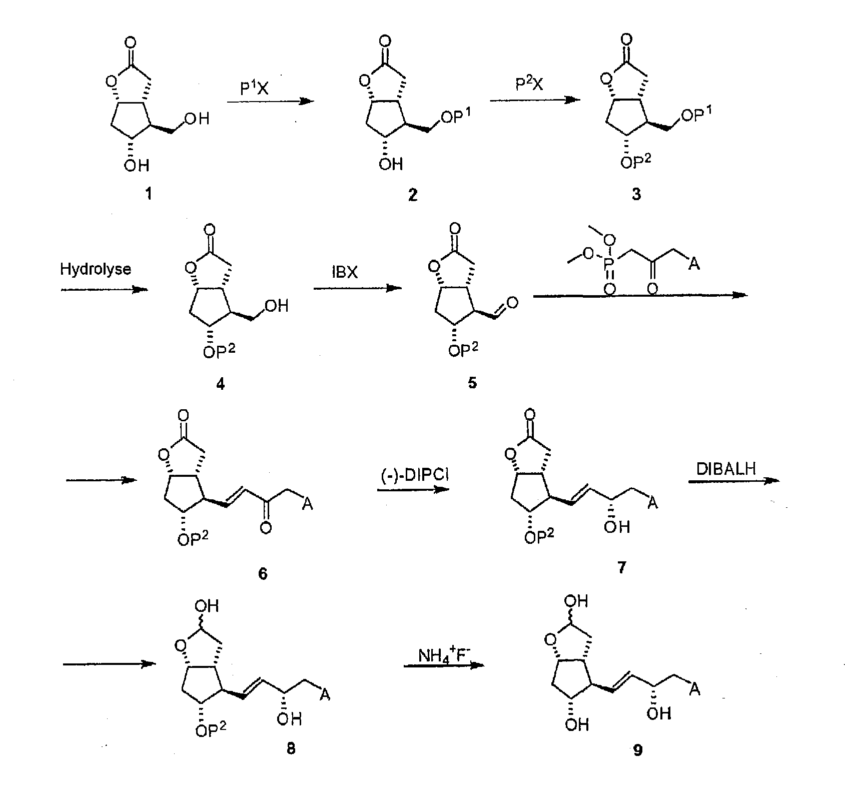 worin
P¹ = Triarylmethyl, gegebenenfalls substituiert an dem Arylrest; -SiQ¹₃, worin Q¹ unabhängig voneinander (C₁-C₄)Alkyl, (C₆-C₁₀)Aryl, (C₁-C₄)Alkyl(C₆-C₁₀)aryl oder (C₁-C₄)Alkoxy(C₆-C₁₀)aryl ist
P² = -SiQ²₂Q³, worin Q² = (C₆-C₁₀)Aryl und Q³ = (C₁-C₄)Alkyl unter der Maßgabe, dass P¹ ≠ P² ist
oder P¹ und P² werden ausgewählt aus den nachstehenden Paaren:
P¹ = *tert*-Butyldimethylsilyl- und P² = *tert*-Butyldiphenylsilyl-
P¹ = Triphenylmethyl- und P² = *tert*-Butyldiphenylsilyl-
P¹ = Triphenylmethyl- und P² = Triphenylsilyl-
X = Halogen, -OSO₂CF₃
A = Benzyl oder (3-Trifluormethyl)benzyloxy
(-)-DIPCl = (-)-Diisopinocampheylboran
DIBALH = Diisobutylaluminiumhydrid
IBX = 2-Iodoxybenzoesäure.

2. Verfahren nach Anspruch 1, wobei die Überführung von Verbindung **1** in Verbindung **3** mit P¹ = *tert*-Butyldimethylsilyl-und P² = *tert*-Butyldiphenylsilyl- ein Ein-Topf-Verfahren ist, ausgeführt in *N*,*N*-Dimethylformamid als ein Lösungsmittel durch Umsetzung von zunächst mit *tert*-Butyldimethylsilylchlorid und Imidazol bei einer Temperatur zwischen -15 und 5°C bis zur vollständigen Überführung in **2**, gefolgt von der direkten Zugabe in das Reaktionsgemisch eines zweiten Teils von Imidazol und *tert*-Butyldiphenylsilylchlorid bei einer Temperatur zwischen 0°C und 25°C.

3. Verfahren nach Anspruch 1, wobei die Oxidation der Verbindung **4** zur Verbindung **5** durchgeführt wird mit IBX bei einer Temperatur im Bereich von 0°C bis 40°C bei einem Molverhältnis von IBX und Verbindung **4** im Bereich von 1 bis 4 in einem Lösungsmittel ausgewählt aus Dimethylsulfoxid, *N*,*N-*Dimethylformamid, Tetrahydrofuran, Methyltetrahydrofuran oder einem Gemisch davon.

4. Verfahren nach den Ansprüchen 1-3, wobei Verbindung **9a**, entsprechend der Verbindung **9** in der A = Benzyl, überführt wird in Bimatoprost, entsprechend der Verbindung (I), worin R = CH₃CH₂NH-, R' = Benzyl und ---- eine Doppelbindung ist, gemäß den nachfolgenden Schritten:

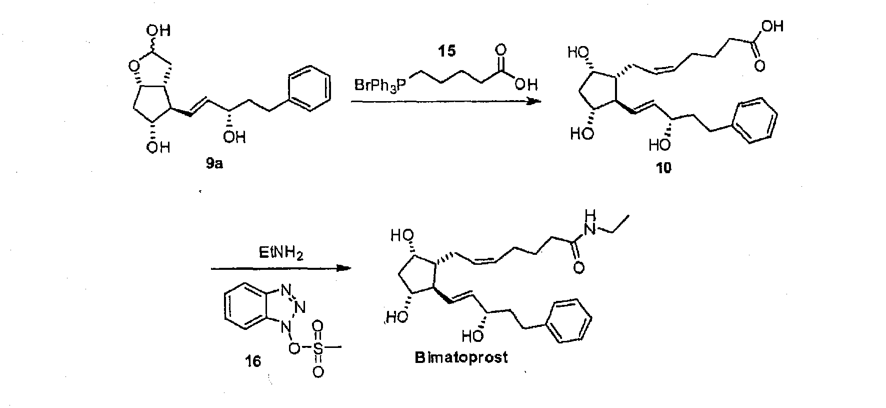

5. Verfahren nach Anspruch 4, wobei die Überführung der Verbindung **10** in Bimatoprost durchgeführt wird durch Umsetzung mit Ethylamin in einem organischen Lösungsmittel bei einer Temperatur zwischen -35 und 25°C in Gegenwart von Triethylamin und einem geeigneten Kupplungsreagens.

6. Verfahren nach Anspruch 5, wobei das Molverhältnis der Verbindung **10** und Ethylamin zwischen 1 und 5 liegt.

7. Verfahren nach Anspruch 6, wobei das Molverhältnis 3,5 beträgt.

8. Verfahren nach Anspruch 5, wobei das Kupplungsreagens 1-(Methylsulfonyloxy)benzotriazol ist.

9. Verfahren nach Anspruch 5, wobei das Molverhältnis von Verbindung 10 und dem Kupplungsreagens zwischen 1 und 2,5 liegt.

10. Verfahren nach Anspruch 9, wobei das Molverhältnis 1,8 beträgt.

11. Verfahren nach Anspruch 5, wobei das organische Lösungsmittel ausgewählt ist aus Amiden, Ethern, Ketonen und chlorierten Lösungsmitteln.

12. Verfahren nach Anspruch 11, wobei das organische Lösungsmittel *N*,*N*-Dimethylformamid ist.

13. Verfahren nach Anspruch 5, wobei die Umsetzung durchgeführt wird bei einer Temperatur zwischen -20°C und -10°C in Gegenwart von Triethylamin.

14. Verfahren nach den Ansprüchen 1-3, wobei Verbindung 9a, entsprechend der Verbindung **9**, in der A = Benzyl ist, überführt wird in Latanoprost, entsprechend der Verbindung (I), worin R = (CH₃)₂CHO-, R' = Benzyl und ---- eine Einfachbindung ist, gemäß den nachfolgenden Schritten:

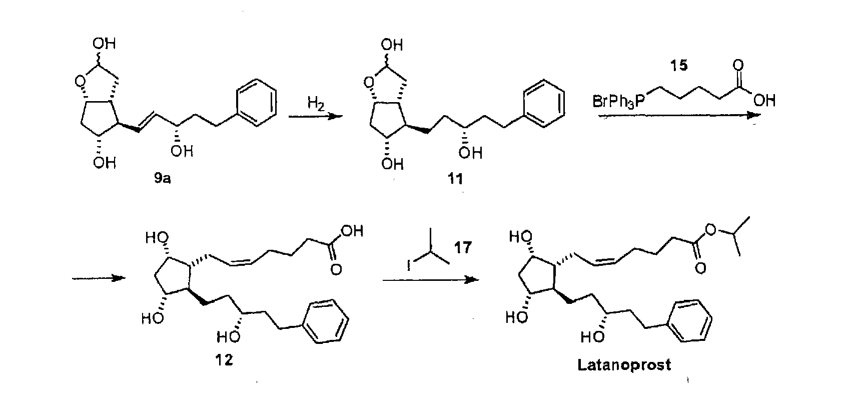

15. Verfahren nach den Ansprüchen 1-3, wobei Verbindung **9b**, entsprechend der Verbindung **9**, in der A = (3-Trifluormethyl)benzyloxy ist, überführt wird in Travoprost, entsprechend der Verbindung (**I**), worin R = (CH₃)₂CHO-, R' = (3-Tri-fluormethyl)benzyloxy und ---- eine Doppelbindung ist, gemäß den nachfolgenden Schritten:

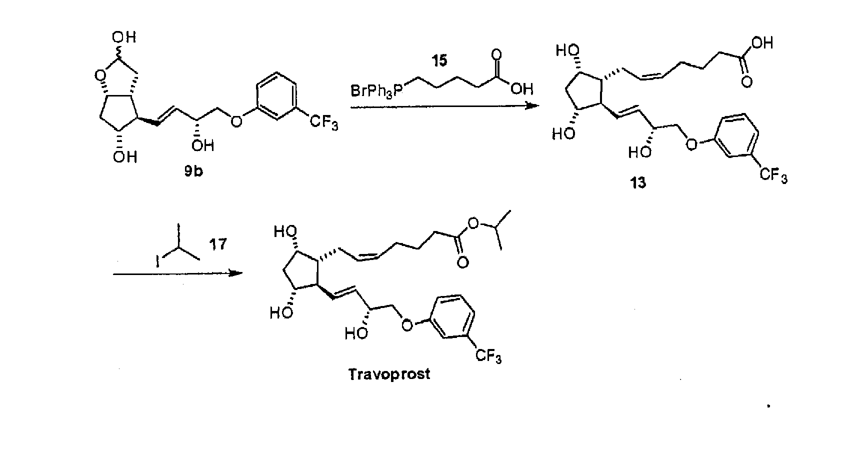

## Revendications

1. Procédé pour la préparation de dérivés de PGF_{2α} répondant à la formule (I) 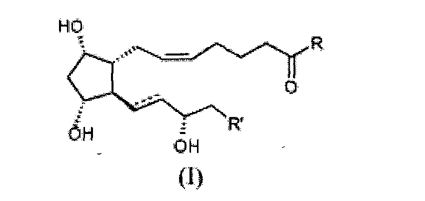 dans laquelle :
1) R représente un groupe (CH₃)₂CHO-, R' représente un groupe benzyle et le signe ---- dans la formule (I) représente une liaison simple ;
2) R représente un groupe (CH₃)₂CHO-, R' représente un groupe (3-trifluorométhyl)benzyloxy et le signe ---- dans la formule (I) représente soit une liaison simple, soit une liaison double ;
3) R représente un groupe CH₃CH₂NH-, R' représente un groupe benzyle et le signe ---- dans la formule (I) représente soit une liaison simple, soit une liaison double ;
conformément au schéma réactionnel indiqué ci-après : 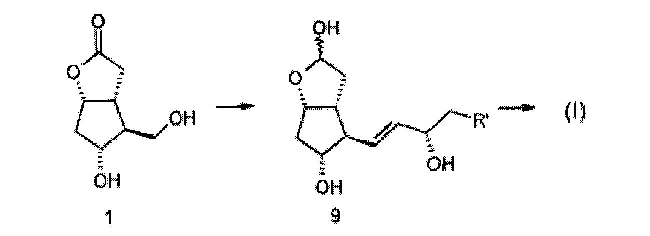 **caractérisé en ce qu'**on transforme le composé 1 en composé 9 en passant par les étapes suivantes : 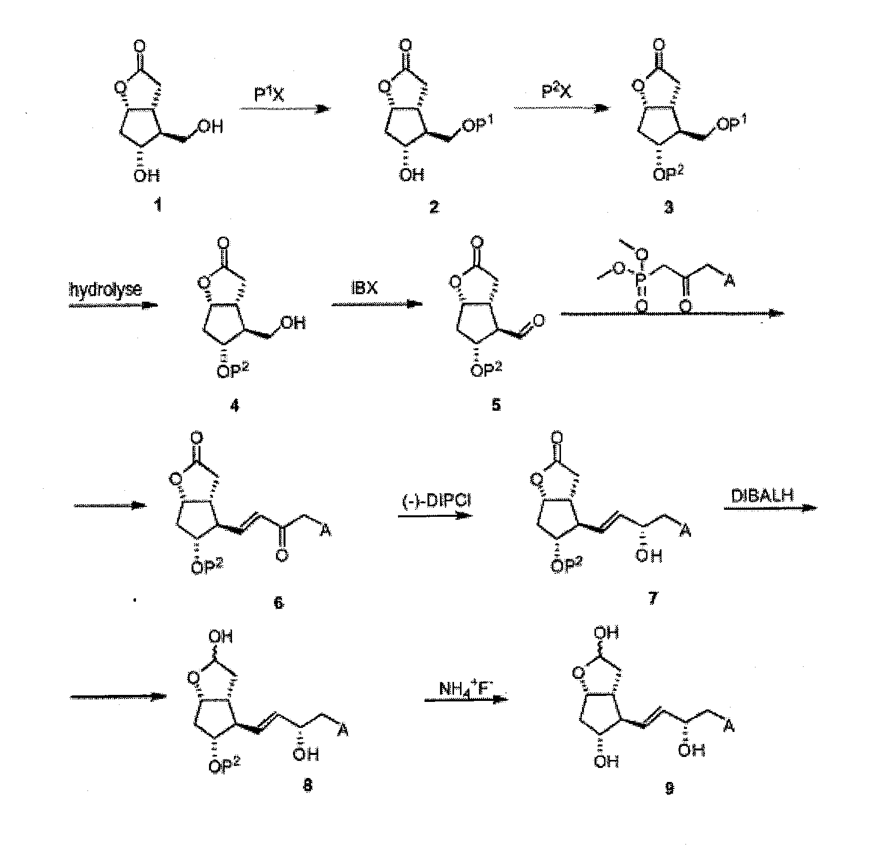 dans lesquelles :
P¹ représente un groupe triarylméthyle substitué de manière facultative sur la fraction aryle ; un groupe -SiQ¹₃ dans lequel Q¹ représentent indépendamment l'un de l'autre un groupe alkyle en C₁-C₄, un groupe aryle en C₆-C₁₀, un groupe alkyl(en C₁-C₄)aryle en C₆-C₁₀ ou un groupe alcoxy(en C₁-C₄)aryle en C₆-C₁₀ ;
P² représente un groupe -SiQ²₂Q³ dans lequel Q² représente un groupe aryle en C₆-C₁₀ et Q³ représente un groupe alkyle en C₁-C₄ ; avec cette condition que P¹ ≠ P² ;
ou bien P¹ et P² sont choisis parmi les paires indiquées ci-après :
P¹ représente un groupe tert-butyldiméthylsilyle et P² représente un groupe tert-butyldiphénylsilyle ;
P¹ représente un groupe triphénylméthyle et P² représente un groupe tert-butyldiphénylsilyle ;
P¹ représente un groupe triphénylméthyle et P² représente un groupe triphénylsilyle ;
X représente un atome d'halogène, un groupe -OSO₂CF₃ ;
A représente un groupe benzyle ou un groupe (3-trifluorométhyl)benzyloxy ;
(-)-DIPCI représente le (-)diisopinocamphénylborane ;
DIBALH représente l'hydrure de diisobutylaluminium ;
IBX représente l'acide 2-iodoxybenzoïque.

2. Procédé selon la revendication 1, dans lequel la conversion du composé 1 en composé 3 dans lequel P¹ représente un groupe tert-butyldiméthylsilyle et P² représente un groupe tert-butyldiphénylsilyle est un procédé en un seul pot mis en oeuvre dans du *N*,*N*-diméthylformamide à titre de solvant en procédant d'abord à une mise en réaction avec du chlorure de tert-butyldiméthylsilyle et de l'imidazole à une température entre -15 et 5 °C jusqu'à la conversion complète en **2**, suivie d'une addition directe dans le mélange réactionnel d'une deuxième portion d'imidazole et de chlorure de tert-butyldiphénylsilyle à une température entre 0 °C et 25 °C.

3. Procédé selon la revendication 1, dans lequel l'oxydation du composé **4** pour obtenir le composé **5** est mise en oeuvre avec du IBX à une température qui se situe dans la plage de 0 °C à 40 °C avec un rapport molaire entre le IBX et le composé **4** qui se situe dans la plage de 1 à 4 dans un solvant choisi parmi le diméthylsulfoxyde, le *N*,*N*-diméthylformamide, le tétrahydrofuranne, le méthyltétrahydrofuranne ou un de leurs mélanges.

4. Procédé selon les revendications **1** à 3, dans lequel on transforme le composé **9a**, correspondant au composé **9** dans lequel A représente un groupe benzyle, en Bimatoprost, correspondant au composé (I) dans lequel R représente un groupe CH₃CH₂NH-, R' représente un groupe benzyle et le signe ---- représente une liaison double, conformément aux étapes suivantes :

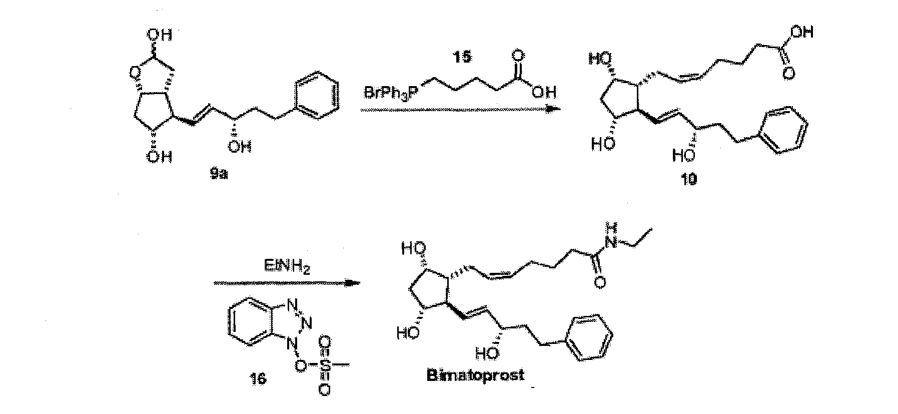

5. Procédé selon la revendication 4, dans lequel on met en oeuvre la conversion du composé **10** en Bimatoprost via mise en une réaction avec de l'éthylamine dans un solvant organique à une température entre -35 et 25 °C en présence de triéthylamine et d'un agent de couplage approprié.

6. Procédé selon la revendication 5, dans lequel le rapport molaire entre le composé 10 et l'éthylamine est compris entre 1 et 5.

7. Procédé selon la revendication 6, dans lequel ledit rapport molaire s'élève à 3,5.

8. Procédé selon la revendication 5, dans lequel le réactif de couplage est le 1-(méthylsulfonyloxy)benzotriazole.

9. Procédé selon la revendication 5, dans lequel le rapport molaire entre le composé 10 et le réactif de couplage est compris entre 1 et 2,5.

10. Procédé selon la revendication 9, dans lequel ledit rapport molaire s'élève à 1,8.

11. Procédé selon la revendication 5, dans lequel le solvant organique est choisi parmi des amides, des éthers, des cétones et des solvants chlorés.

12. Procédé selon la revendication 11, dans lequel le solvant organique est le N,N-diméthylformamide.

13. Procédé selon la revendication 5, dans lequel la réaction est mise en oeuvre à une température comprise entre -20 °C et -10 °C en présence de triéthylamine.

14. Procédé selon les revendications 1 à 3, dans lequel on transforme le composé 9a, correspondant au composé 9 dans lequel A représente un groupe benzyle, en Latanoprost, correspondant au composé (I) dans lequel R représente un groupe (CH₃)₂CHO-, R' représente un groupe benzyle et le signe ---- représente une liaison simple, conformément aux étapes suivantes :

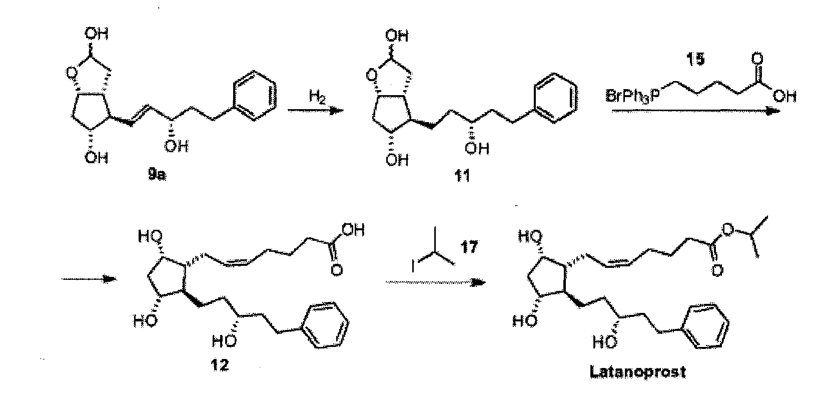

15. Procédé selon les revendications 1 à 3, dans lequel on transforme le composé 9b, correspondant au composé 9 dans lequel A représente un groupe (3-trifluorométhyl)benzyloxy, en Travoprost, correspondant au composé (I) dans lequel R représente un groupe (CH₃)₂CHO-, R' représente un groupe (3-trifluorométhyl)benzyloxy et le signe ---- représente une liaison double, conformément aux étapes suivantes :
